# FASCICULE DE BREVET EUROPEEN

(11) **EP 1 730 296 B1**
(45) Date de publication et mention de la délivrance du brevet: **10.06.2009**
(21) Numéro de dépôt: 05742624.9
(22) Date de dépôt: 22.03.2005
(51) Int. Cl.: C12Q 1/04

(54) **METHODE D'IDENTIFICATION DE FAMILLE DE VOIE METABOLIQUE PAR SELECTION POSITIVE**
VERFAHREN ZUR IDENTIFIZIERUNG EINER STOFFWECHSELFAMILIE MITTELS POSITIVER SELEKTION
METHOD FOR THE IDENTIFICATION OF A METABOLIC PATHWAY FAMILY BY MEANS OF POSITIVE SELECTION

(30) Priorité: 23.03.2004 FR 0402985
(43) Date de publication de la demande: 13.12.2006
(73) Titulaire: Libragen, 31000 Toulouse (FR)
(72) Inventeur: MONSAN, Pierre, F-31700 Mondonville (FR); BENSOUSSAN, Claude, F-31290 Renneville (FR); REULET, Philippe, F-75015 Paris (FR); NALIN, Renaud, F-31650 Auzielle (FR); ROBE, Patrick, F-31450 Odars (FR); TUPHILE, Karine, F-91390 Morsang sur Orge (FR); GILLET, Benjamin, F-69007 Lyon (FR); PUJIC, Pierre, F-69360 Serezin du Rhône (FR)
(74) Mandataire: Gallois, Valérie
(86) Numéro de dépôt international: PCT/FR2005/000696
(87) Numéro de publication internationale: WO 2005/095634

(56) Documents cités:
- WO-A-98/17811
- US-B1- 6 368 793
- RONDON M R ET AL: "CLONING THE SOIL METAGENOME: A STRATEGY FOR ACCESSING THE GENETIC AND FUNCTIONAL DIVERSITY OF UNCULTURED MICROORGANISMS" APPLIED AND ENVIRONMENTAL MICROBIOLOGY, WASHINGTON,DC, US, vol. 66, no. 6, juin 2000 (2000-06), pages 2541-2547, XP000946829 ISSN: 0099-2240

## Description

### Champ de l'invention

L'invention se rapporte à la sélection directe de voies métaboliques ayant une fonction déterminée dans la transformation d'un substrat {A} en un produit cible {B}, présentant un intérêt industriel, chimique, pharmaceutique, cosmétique, agrochimique ou nutraceutique. Plus particulièrement l'invention porte sur la détection, au sein de banques metagénomiques, de nouvelles voies de biosynthèse impliquées dans une réaction biochimique qui conduit au produit {B}. A travers la sélection et la caractérisation de ces nouvelles voies métaboliques, permettant de produire {B} par voie enzymatique, la présente invention offre, soit une alternative à la synthèse chimique de la molécule d'intérêt {B}, soit permet la synthèse de produit {B} jusqu'à présent inaccessible par voie chimique.

### Contexte de l'invention

La biocatalyse, définie comme la synthèse biologique de molécules d'intérêt par voie enzymatique, connaît une popularité croissante en proposant une puissante alternative à la synthèse chimique, en terme de coût, de temps, d'étapes de purification, et de simplicité de mise en oeuvre. La mise en place de tout nouveau procédé de biocatalyse à l'échelle industrielle nécessite toutefois (i) d'identifier l'enzyme (ou les) enzyme(s) permettant de convertir spécifiquement le substrat fourni en le produit recherché, (ii) d'identifier l'enzyme (ou les) enzyme(s) permettant de réaliser la catalyse de façon stable et dans les conditions particulières liées au processus industriel (thermostabilité, pH, tolérance aux conditions dénaturantes de solvants organiques, etc).

De part leur répartition universelle, y compris dans les environnements les plus extrêmes, les microorganismes sont connus pour pouvoir réaliser des fonctions enzymatiques tout à fait originales et dans des conditions compatibles avec les processus industriels évoqués précédemment.

Cependant, l'approche prometteuse d'exploiter ces fonctions bactériennes a toujours été considérablement limitée par un verrou technologique : l'isolement et la *culture in vitro* de l'énorme potentiel que constitue la diversité bactérienne. La plupart des bactéries se développant dans des environnements naturels complexes (sols et sédiments, environnements aquatiques, systèmes digestifs, ...) n'ont pas été cultivées car leurs conditions optimales de culture sont inconnues ou trop difficiles à reproduire. De nombreux travaux scientifiques démontrent cet état de fait, et il est maintenant largement admis que seulement 0.1 à 1% de la diversité bactérienne, tous environnements confondus, ont été isolés et cultivés (Amann et al, 1995, Microb. Rev., 59:143-169). Si la recherche de nouvelles voies biocatalytiques au sein de collections de souches microbiennes a montré son efficacité, elle présente néanmoins l'inconvénient majeur de n'exploiter qu'une part infime de la biodiversité bactérienne.

De nouvelles approches ont été développées pour contourner ce point critique de l'isolement des bactéries et pour accéder à cet énorme potentiel génétique constitué par les systèmes d'adaptation des bactéries à travers leur longue évolution. Cette approche se nomme Metagénomique parce qu'elle concerne un ensemble de génomes d'une communauté bactérienne sans aucune distinction (metagénome).

La metagénomique implique l'extraction directe d'ADN à partir d'échantillons environnementaux, leur propagation et leur expression dans des hôtes bactériens cultivables. La metagénomique au sens strict a tout d'abord été utilisée pour l'identification de nouveau phyla bactériens (Pace, 1997, Science, 276 :734-740). Cette approche est basée sur le clonage spécifique de gènes reconnus pour leur intérêt phylogénétique, comme par exemple ADNr 16S. D'autres développements ont été réalisés pour l'identification de nouvelles enzymes d'intérêt environnemental ou industriel (Brevet Terragen Diversity N° US 6,441,148). Dans ces deux approches, la metagénomique commence par une sélection des gènes recherchés. Cette sélection se fait par une approche PCR (Polymerase Chain Reaction), généralement avant l'étape de clonage. Dans le dernier cas, le vecteur de clonage est préférentiellement un vecteur d'expression (*i.e*. il contient des séquences régulatrices en amont du fragment d'ADN cloné, lui permettant de faire exprimer l'ADN cloné dans un hôte d'expression donné).

Des développements plus récents considèrent la totalité du metagénome. Ainsi aucune sélection ni aucune identification ne sont effectuées avant la réalisation de la banque d'ADN metagénomique, de manière tout à fait aléatoire. Cette approche donne donc accès à la totalité du potentiel génétique de la communauté bactérienne prospectée sans aucun *a priori.*

De manière générale, les bactéries ont un rôle important dans le fonctionnement des écosystèmes. De fait, elles sont quantitativement bien représentées. Par exemple, on estime qu'un gramme de sol peut contenir de 1 000 à 10 000 espèces de bactéries différentes avec 10⁷ à 10⁹ cellules en considérant les bactéries cultivables et non-cultivables. Récupérer l'ensemble de cette diversité dans des banques d'ADN metagénomique demande de pouvoir générer et gérer un grand nombre de clones.

Dans cette dernière approche, les banques d'ADN sont composées de plusieurs dizaines, centaines de milliers, voir même de plusieurs millions de clones recombinants qui se différencient les uns des autres par l'ADN qu'ils ont incorporé. A ce titre, la taille moyenne des inserts metagénomiques clonés est de toute première importance dans la recherche de voies de biosynthèse bactériennes car la plupart du temps ces voies sont organisées en cluster chez les bactéries. Plus les fragments d'ADN clonés sont grands (supérieur à 30Kb), plus on limite le nombre de clones à analyser et plus on a de chance de récupérer des voies métaboliques complètes, permettant d'obtenir la conversion d'un substrat {A} en un produit cible {B} et en source de croissance.

Etant donné le nombre important de clones recombinants à étudier et le nombre d'essais à réaliser, de nombreux laboratoires s'orientent vers l'utilisation de systèmes d'hybridation à haute densité (membranes à haute densité ou puces à ADN), notamment dans la caractérisation des communautés bactériennes (pour revue voir Zhou et al., 2003, Curr. Opin. Microbial., 6 :288-294).

Même si l'ensemble de ces données ne portent pas sur des banques metagénomiques, elles fournissent néanmoins un grand nombre d'informations telles que la quantification de différents gènes fonctionnels (Cho *et al.,* 2003), l'étude de gènes fonctionnels et leur diversité (Wu et al., 2001, Appl. Environ. Microbiol., 67 :5780-5790) et la détection directe de gènes ADNr 16S (Small *et al.,* 2001). Seule une étude relate l'utilisation de la metagénomique en combinaison avec les puces à ADN (Sebat et al., 2003, Appl. Environ. Microbiol., 69: 4927-4934) pour l'identification de clones contenant de l'ADN issu de bactéries non-cultivables et leur sélection pour des analyses supplémentaires.

Le criblage d'activités enzymatiques ou d'activités antibactériennes à partir de banques metagénomiques a été largement décrit dans la littérature scientifique. Les études ont porté, par exemple, sur la détection directe d'activité chitinase (Cottrell et al., 1999, Appl. Environ. Microbiol., 65 :2553-2557), lipase (Henne et al., 2000, Appl. Environ. Microbiol., 66 :3113-3116), DNAse, et amylase (Rondon et al., 2000, Appl. Environ. Microbiol., 66 :2541-2547) etc... Dans ces études, les bactéries hôtes contenant les clones recombinants sont mises en culture sur un milieu complémenté par le substrat dont on cherche la métabolisation, et le criblage de l'activité est généralement basé sur la manifestation de halos ou de précipitats autours des colonies, ou par un changement d'aspect des colonies qui métabolisent le substrat étudié. Il est à noter que les activités enzymatiques détectées à travers ces exemples constituent des activités nouvelles pour la bactérie hôte mais ne sont pas indispensables à la croissance de celle-ci dans les exemples présentés. Une approche similaire a été décrite dans le brevet (Chromaxome N° 5,783,431). Ce brevet décrit une méthode de criblage d'activité basée sur l'encapsulation de clones individuels ou poolés d'une banque dans une matrice stable, inerte et poreuse (avantageusement de l'alginate), sous la forme de macro- ou de micro-goutelettes. Les gouttelettes sont par exemple soumises à une culture liquide contenant les éléments nutritifs nécessaires à la croissance bactérienne ainsi qu'un substrat (par exemple X-glucosaminide, X-acetate, X-glucopyranoside, etc..) dont la métabolisation se traduit par l'apparition d'une coloration bleue.

De manière alternative, le criblage phénotypique décrit dans le Brevet Proteus (N° FR 2 786 788) s'appuie sur une préparation préalable des séquences d'acides nucléiques codant pour la protéine cible (éléments amont et aval nécessaires à la transcription et à la traduction des gènes cibles), la transcription et la traduction *in vivo,* puis la détection et la mesure de l'activité des protéines cibles.

Toutes ces méthodes de criblage imposent l'utilisation de systèmes à haut débit car ils impliquent le passage de tous les clones à travers le test de criblage pour identifier les clones d'intérêt répondant positivement aux tests. A ce titre, la société Diversa, leader dans le domaine de la découverte de nouvelles molécules, a développé une plateforme unique, appelée GigaMatrix, permettant un criblage à ultra haut débit, de l'ordre de 1 milliard de clones par jours (http://www.diversa.com/techplat/gigamatrix/default.asp).

Une autre approche a été déjà décrite dans le brevet WO 00/22170 de Microgenomics (N° US 6,368,793 B1). Ce brevet décrit une méthodologie d'identification de voie métabolique transformant un substrat S en un produit recherché T par création ou identification d'un organisme génétiquement manipulé dont la capacité a réaliser cette réaction est placée sous le contrôle d'un promoteur inductible. Cet organisme est utilisé pour cribler des fragments d'acides nucléiques pour détecter un gène impliqué dans la transformation d'un substrat en produit. La mise en oeuvre de cette méthode, impose l'identification et la caractérisation génétique des gènes responsables de la dégradation de T chez l'hôte d'expression pour qu'ils puissent être mis sous contrôle d'un promoteur inductible. Une telle construction n'est pas toujours envisageable, en particulier quand les gènes d'intérêt sont disséminés sur le génome et les risques « de fuite » à l'inducteur sont possibles. D'autre part, elle représente des travaux extrêmement lourds qu'il faudra renouveler pour chaque étude d'un produit T. Enfin, dans cette approche, l'organisme utilisé doit être capable d'incorporer et de métaboliser S et T. L'ensemble des éléments énoncés démontre les limites de l'efficacité d'une telle approche.

La majorité de ces technologies, à l'exception de celle décrite par Microgenomics, imposent donc l'organisation préalable des banques, *i.e*. l'individualisation, le stockage et la conservation des clones dans des formats compatibles avec les systèmes de criblage mentionnés ci-dessus. Par ailleurs, l'adéquation d'une banque metagénomique à une problématique donnée (par exemple la recherche d'une fonction enzymatique spécifique) ne peut être établie que lorsque l'intégralité des clones composant cette banque ont été soumis au crible. Plusieurs centaines de milliers de clones doivent souvent être criblés pour ne détecter peut-être qu'un seul clone d'intérêt. La réalisation d'une banque metagénomique est en effet assujetie à un certain nombre de contraintes, telles que le choix préalable de l'environnement prospecté, de la (ou les) communauté(s) bactériennes visée(s) au sein de cet environnement, du vecteur de clonage ou d'expression, des tailles d'inserts clonés, et de l'organisme hôte susceptible d'exprimer au mieux l'ADN metagénomique hétérologue.

Le temps et les moyens mis en oeuvre lors de la réalisation de la banque metagénomique puis son criblage sont donc clés, avec un faible espoir de succès. L'augmentation des chances de découverte impliquerait, dans l'absolu, la réalisation d'une banque metagénomique propre à chaque problématique, pour répondre au mieux aux objectifs posés.

### Résumé de l'invention

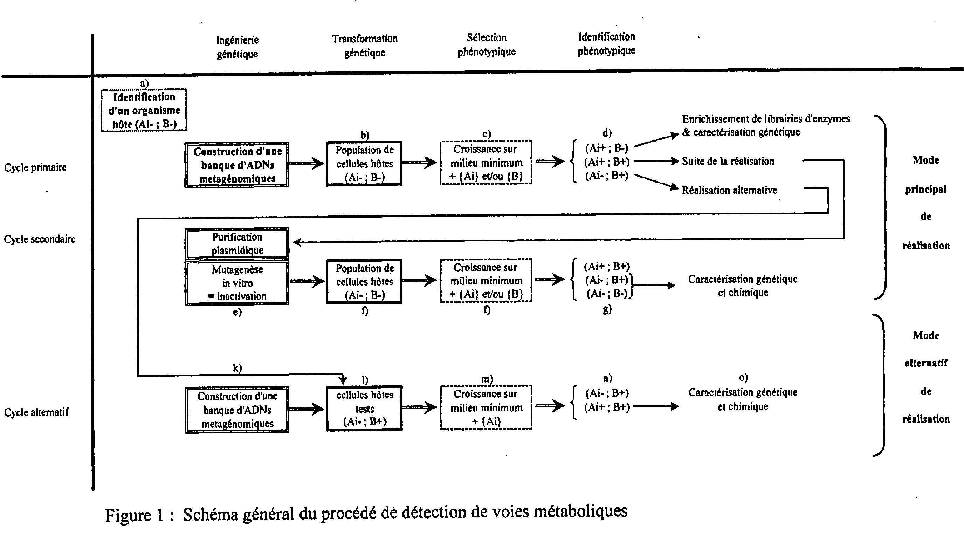

La présente invention concerne une méthode d'identification d'une voie métabolique ou de familles de voies métaboliques permettant la transformation d'un ou plusieurs substrat(s) {Ai} en un produit recherché {B}. Cette méthode est fondée sur la sélection ou la préparation de cellules comprenant au moins une voie métabolique ou une famille de voies métaboliques permettant la transformation d'un ou plusieurs substrat(s) {Ai} en un produit recherché {B}. Elle permet en outre l'identification et la caractérisation du ou des gènes codant pour l'enzyme ou les enzymes impliqué(s) dans la conversion du substrat {Ai} en produit {B}. La présente invention, basée sur une succession de cycles de transformation-sélection-purification, cible l'énorme potentiel microbien (Figure 1). Cette méthode comprend les étapes suivantes :
a) fournir une population de cellules hôtes (Ai- ; B-) incapables de métaboliser ledit ou lesdits substrats {Ai} et ledit produit {B} ;
b) transformer ladite population de cellules hôtes avec une banque de séquences d'acides nucléiques ;
c) tester en parallèle ladite population de cellules hôtes transformées sur des milieux minimum contenant soit un des substrat(s) {Ai}, soit ledit produit {B} comme seule source d'un élément essentiel à la croissance ; et,
d) sélectionner ladite ou lesdites cellule(s) hôte(s) transformées capable(s) de croissance sur un milieu minimum contenant un des substrat(s) {Ai} et sur un milieu minimum contenant ledit produit {B} (Ai+ ; B+) puis facultativement isoler la molécule d'acide nucléique introduite lors de la transformation à l'étape b) et conférant le phénotype (Ai+ ; B+).

De préférence, la méthode comprend, avant l'étape c), une étape consistant à tester la population de cellules hôtes transformées sur un milieu minimum contenant le(s) substrat(s) {Ai} et ledit produit {B} comme seule source d'un élément essentiel à la croissance et sélectionner ladite ou lesdites cellule(s) hôte(s) transformées capable(s) de croissance sur ledit milieu minimum contenant le(s) substrat(s) {Ai} et ledit produit {B}; ladite ou lesdites cellule(s) hôte(s) sélectionnée(s) étant ensuite soumise à l'étape c) et suivantes.

La méthode selon la présente invention peut comprendre également, après l'étape d), les étapes suivantes :
e) effectuer une mutagenèse *in vitro,* où toutes autres méthodes connues de l'homme de l'art permettant de conduire à un même résultat, de la molécule d'acide nucléique isolée de ladite ou lesdites cellule(s) hôte(s) transformées (Ai+ ; B+) à l'étape d) ;
f) re-transformer la population de cellules hôte (Ai- ; B-) décrite à l'étape a) avec la population des acides nucléiques mutés *in vitro* à l'étape e) et tester les cellule(s) hôte(s) ainsi transformées sur des milieux minimum contenant soit un des substrat(s) {Ai}, soit ledit produit {B} comme seule source d'un élément essentiel à la croissance; et,
g) sélectionner ladite ou lesdites cellule(s) hôte(s) transformées incapable(s) de croissance sur un milieu minimum contenant un des substrat(s) {Ai} et capable(s) de croissance sur un milieu minimum contenant ledit produit {B} (Ai- ; B+) puis facultativement isoler la molécule d'acide nucléique mutée.

De plus, la méthode peut comprendre la caractérisation du ou des gènes codant pour l'enzyme ou les enzymes impliqué(s) dans la conversion du substrat {Ai} en produit {B} et isolés de ladite ou lesdites cellule(s) hôte(s) transformée(s) (Ai- ; B+) sélectionnée(s) à l'étape g).

Dans une première alternative, la méthode comprend, après l'étape f), à la place ou en parallèle de l'étape g) :
h) sélectionner ladite ou lesdites cellule(s) hôte(s) transformée(s) et devenue(s) incapable(s) de croissance sur un milieu minimum contenant un des substrat(s) {Ai} et sur un milieu minimum contenant ledit produit {B} (Ai- ; B-) ;
i) effectuer une analyse quantitative de l'accumulation du produit {B} de ladite ou lesdites cellule(s) hôte(s) transformée(s) (Ai- ; B-) sur un milieu riche supplémenté en {Ai} ; et
j) sélectionner ladite ou lesdites cellule(s) hôte(s) transformées (Ai- ; B-) accumulant le produit {B} sur un milieu riche et facultativement isoler en parallèle la molécule d'acide nucléique mutée introduite lors de la transformation de l'étape f).

De plus, la méthode peut comprendre la caractérisation du ou des gènes codant pour l'enzyme ou les enzymes impliqué(s) dans la conversion du substrat {Ai} en produit {B} isolés de ladite ou lesdites cellule(s) hôte(s) transformée(s) (Ai- ; B-) sélectionnée(s) à l'étape j).

Dans une seconde alternative, la méthode comprend, après l'étape c), à la place ou en parallèle de l'étape d) et suivantes, les étapes suivantes :
k) sélectionner ladite ou lesdites cellule(s) hôte(s) transformée(s), incapable(s) de croissance sur un milieu minimum contenant un des substrat(s) {Ai} et capable(s) de croissance sur un milieu minimum contenant ledit produit {B}, appelée(s) cellule(s) receveuse(s) (Ai- ; B+) ;
1) transformer ladite ou lesdites cellule(s) receveuse(s) (Ai- ; B+) avec une banque de séquences d'acide nucléique;
m) tester en parallèle ladite ou lesdites cellule(s) receveuse(s) transformée(s) (Ai- ; B+) sur un milieu minimum contenant un des substrat(s) {Ai} ;
n) sélectionner ladite ou lesdites cellule(s) receveuse(s) transformée(s) capable(s) de croissance sur un milieu minimum contenant un des substrat(s) {Ai } ; et
o) caractériser le ou les gènes codant pour l'enzyme ou les enzymes impliqué(s) dans la conversion du substrat {Ai} en produit {B} et isolé(s) de ladite ou lesdites cellule(s) receveuse(s) transformées (Ai+ ; B+) sélectionnée(s) à l'étape n).

Ladite banque de séquences utilisée à l'étape m) peut être la même que celle utilisée à l'étape b) ou être une banque distincte. Si ladite banque de séquence est (i) la même que celle utilisée à l'étape b), c'est à dire que le marqueur de sélection (résistance à un antibiotique, ou marqueur d'auxothrophie) est le même que celui présent chez la (les) cellule(s) receveuse(s) de phénotype (Ai- ; B+), ou est (ii) différente de celle utilisée à l'étape b) mais qu'elle porte néanmoins sur le même marqueur de sélection, alors il est nécessaire de modifier le marqueur de sélection de la séquence d'acide nucleique conférant le phénotype (Ai- ; B+), comme décrit aux étapes kk) à kkkkk). Ladite modification, reposant avantageusement sur le remplacement de la résistance initiale à un antibiotique par une résistance à un second antibiotique, permettra d'appliquer à l'étape m) une double pression de sélection permettant de sélectionner les cellules transformées contenant les deux séquences d'acides nucléiques : la séquence d'acides nucleiques présente initialement et conférant la capacité de croître sur {B} et la séquence d'acides nucléique nouvellement acquise et conférant la capacité de convertir {Ai} en {B}.

De préférence, la méthode comprend, avant l'étape m), tester ladite ou lesdites cellule(s) hôte(s) (Ai- ; B+) transformée(s) sur un milieu minimum contenant plusieurs substrat(s) {Ai} comme seule source d'un élément essentiel à la croissance et sélectionner ladite ou lesdites cellule(s) hôte(s) capable(s) de croissance sur ledit milieu minimum contenant plusieurs substrat(s) {Ai}; ladite ou lesdites cellule(s) hôte(s) sélectionnée(s) étant ensuite soumise(s) à l'étape m) et suivantes.

Dans cette seconde alternative, l'invention concerne également une méthode dans laquelle :
- entre les étapes k) et 1), ladite ou lesdites cellule(s) hôte(s) (Ai- ; B+) est/sont modifiée(s) par le remplacement du premier marqueur de sélection présent dans le vecteur contenant la séquence d'acide nucléique introduite à l'étape b) par un nouveau marqueur de sélection ;
- ladite banque de séquences d'acide nucléique de l'étape 1) comprend un marqueur de sélection différent de celui porté par ladite ou lesdites cellule(s) hôte(s) (Ai- ; B+)
- la méthode comprend en outre les étapes suivantes :
   kk) l'extraction et la purification des vecteurs contenus dans ladite ou lesdites cellule(s) hôte(s) sélectionnée(s) à l'étape k) ;
   kkk) la mutagenèse *in vitro* dudit vecteur purifié à l'étape kk), avantageusement par transposition avec un élément transposable portant par exemple une résistance fonctionnelle à un antibiotique différente de celle pré-existente sur ce vecteur.
   kkkk) la transformation de ladite ou lesdites cellule(s) hôte(s) (Ai- ; B-) incapable(s) de métaboliser ledit ou lesdits substrats {Ai} et ledit produit {B} par les acides nucleiques mutés obtenus à l'étape précédente ;
   kkkkk) la sélection des cellules hôtes transformées contenant uniquement ledit deuxième marqueur de sélection ; ces cellules transformées, de phénotype (Ai- ; B+), appelées formellement cellules receveuses, font alors l'objet de la transformation décrite en l'étape 1).

Lesdites cellules hôtes sont des cellules eucaryotes ou procaryotes. De préférence, elles sont :
- cultivables dans des conditions standards connues de l'homme de l'art,
- transformables ou compétentes et
- capables de maintenir de façon stable les ADN exogènes transformants.

Dans un mode de réalisation préféré, lesdites cellules hôtes sont des bactéries.

Ladite banque de séquences d'acide nucléique peut être une banque metagénomique. Dans un premier mode de réalisation, ladite banque de séquences d'acide nucléique est issue d'organismes procaryotes ou eucaryotes cultivables. Dans un deuxième mode de réalisation, ladite banque de séquences d'acide nucléique est issue d'organismes procaryotes ou eucaryotes non-cultivables.

Dans un mode de réalisation préféré de l'invention, l'élément essentiel à la croissance est le carbone.

De préférence, le marqueur de sélection est un gène de résistance à un antibiotique.

L'invention concerne également les cellules hôtes sélectionnées par les méthodes selon la présente invention, ainsi que leur utilisation, notamment dans des bio-procédés mettant en oeuvre ces cellules hôtes capables de transformer un ou plusieurs substrat(s) {Ai} en un produit recherché {B}. Plus particulièrement, la présente invention concerne l'utilisation d'une cellule hôte sélectionnée à l'étape g), j) ou n) des méthodes selon la présente invention dans un procédé de préparation du produit {B} à partir du substrat {Ai}.

L'invention concerne également le ou les gènes codant pour l'enzyme ou les enzymes impliqué(s) dans la conversion du substrat {Ai} en produit {B} identifiés par les méthodes de la présente invention, un vecteur le ou les contenant, une cellule transformée le ou les contenant, ainsi que toute utilisation de ceux-ci. Plus particulièrement, la présente invention concerne l'utilisation d'une cellule hôte transformée avec le ou les gènes codant pour l'enzyme ou les enzymes impliqué(s) dans la conversion du substrat {Ai} en produit {B} caractérisé(s) selon l'une des méthodes selon la présente invention dans un procédé de préparation du produit {B} à partir du substrat {Ai}.

Enfin, la présente invention concerne une méthode d'identification, de sélection, ou de préparation de cellule hôte (Ai- ; B-) incapable de métaboliser ledit ou lesdits substrats {Ai} et ledit produit {B} comprenant les étapes suivantes :
- tester une population de cellules hôtes, cultivables dans des conditions standards de laboratoire et dans des conditions industrielles de production, transformables, et capables de maintenir de façon stable les ADN exogènes transformants, sur un milieu minimum contenant le(s) substrat(s) {Ai} et ledit produit {B} comme seule source d'un élément essentiel à la croissance; et,
- sélectionner la ou les cellule(s) hôte(s) incapable(s) de croissance sur ledit milieu minimum contenant le(s) substrat(s) {Ai} et ledit produit {B}.

La cellule hôte peut être un cellule procaryote ou eucaryote, de préférence une bactérie.

L'invention concerne également une cellule hôte cultivable dans des conditions standards connues de l'homme de l'art, transformable ou compétente, capable de maintenir de façon stable les ADN exogènes transformants, et incapable de croissance sur ledit milieu minimum contenant le(s) substrat(s) {Ai} et ledit produit {B} comme seule source d'un élément essentiel à la croissance, notamment une cellule hôte obtenue par le procédé indiqué ci-dessus. Elle concerne en outre l'utilisation d'une telle cellule hôte pour l'identification d'au moins une voie métabolique ou une famille de voies métaboliques permettant la transformation d'un ou plusieurs substrat(s) {Ai} en un produit recherché {B}.
i est un entier de 1 à n, plus particuliérement de 1 à 100, de préférence de 1 à 50 ou de 1 à 10.

### Description des figures

Figure 1 : Schéma général du procédé de détection de voies métaboliques.
Figure 2 : Schéma du cycle primaire de transformation-sélection.
Figure 3 : Schéma du cycle secondaire de transformation-sélection.
Figure 4 et Figure 5: Schéma du cycle secondaire alternatif de sélection.

### Description détaillée de l'invention

La présente invention propose de sélectionner et d'identifier une voie métabolique permettant la transformation un substrat {Ai} en un produit {B}, ne dépendant pas de la création/identification d'un organisme capable de métaboliser {B} sous un signal inductible en composé essentiel et ne dépendant pas non plus de la capacité à incorporer le produit recherché {B}. L'invention permet d'exploiter spécifiquement des voies métaboliques provenant d'organismes capables de produire le produit cible {B} mais également capables de le cataboliser. De fait, l'invention exploitant spécifiquement les voies métaboliques permettant de convertir un substrat {Ai} en un produit {B}, elle permet de s'affranchir de toutes les voies connexes de catabolisme du produit {B}, susceptibles d'affecter l'accumulation de {B} chez l'organisme d'origine.

L'invention propose de réduire considérablement le temps et les coûts associés à la recherche d'une nouvelle fonction enzymatique au sein de banques metagénomiques, en rendant la fonction recherchée directement détectable par sélection positive. De manière préférentielle, la fonction recherchée est indispensable à la survie de la cellule recombinée. La détection de cette voie métabolique, conduisant de façon non exclusive à la transformation d'un substrat {Ai} en un produit cible {B}, implique donc que le composé {B} soit, directement ou indirectement, strictement requis pour la croissance de la cellule hôte. L'invention se distingue *de facto* d'un procédé de recherche de complémentation qui viserait à détecter des voies métaboliques permettant à la cellule hôte de croître sur {Ai}.

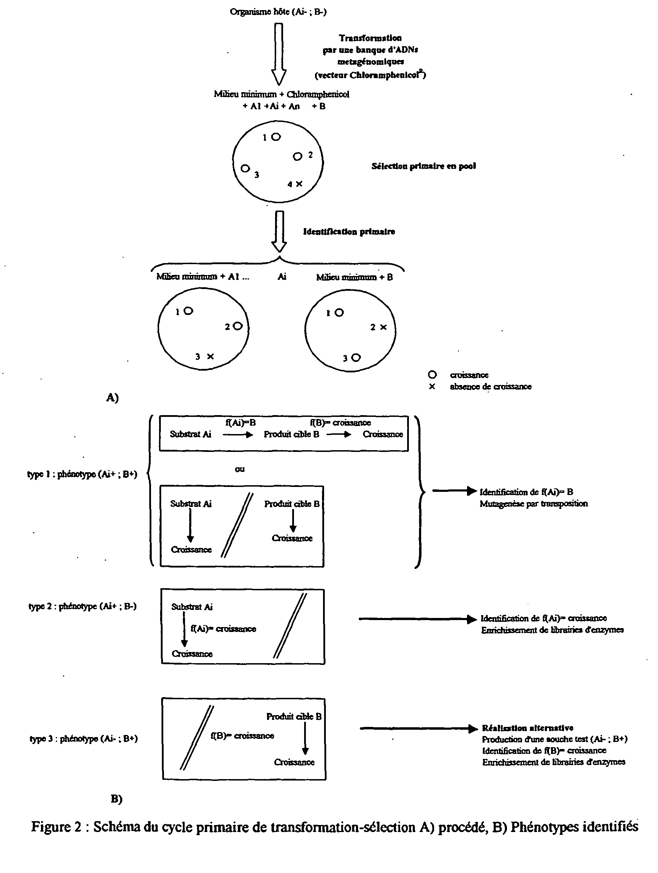

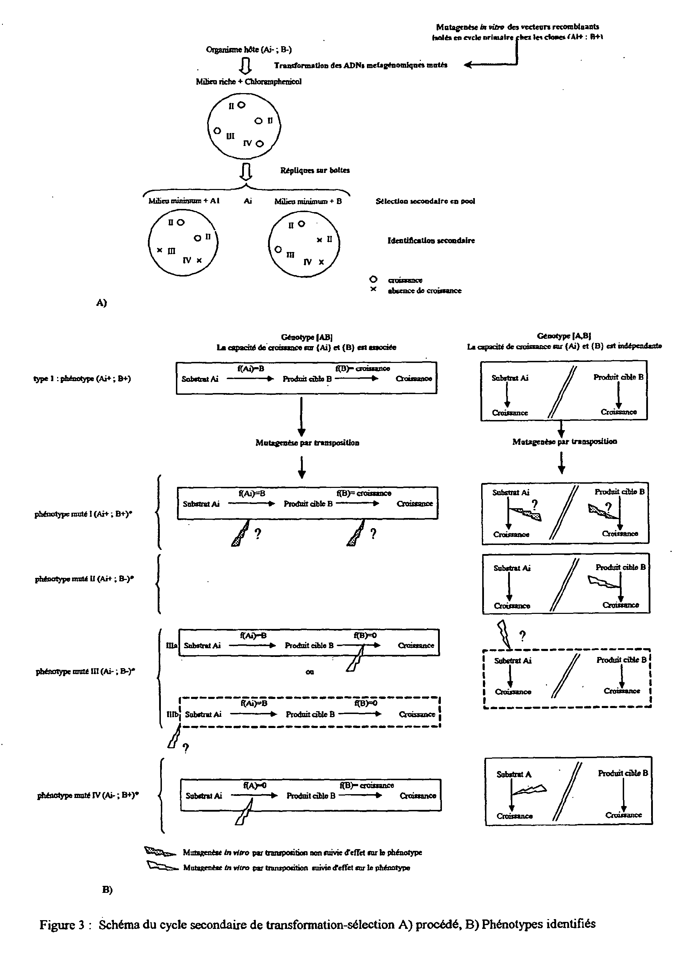

Le procédé de l'invention comprend dans un premier mode de réalisation un cycle primaire (figure 2) de sélection-transformation comprenant les étapes suivantes:
A- L'identification d'une souche hôte, de préférence bactérienne, incapable de se développer sur un (ou plusieurs) substrat(s) {Ai} (i de 1 à n) et sur le produit {B} de la fonction recherchée ;
B- La transformation de ladite souche identifiée par une banque de séquences d'acide nucléique, de préférence d'ADN environnemental, clonée dans un vecteur approprié;
C- La sélection primaire de clones recombinants sur milieu minimum contenant le(s) substrat(s) {Ai} et le produit cible {B} comme seules sources d'un élément essentiel à la croissance (figure 1). Les clones recombinants capables de métaboliser au moins un des précursseurs fournis (un ou plusieurs substrats {Ai} et/ou le produit cible {B}) sont conservés puis testés parallèlement sur milieu minimum contenant seulement un des précursseurs. Cette sélection primaire permet donc de sélectionner en une étape trois types de phénotype :
   - type 1 : des clones recombinants capables de croître à la fois sur un (ou plusieurs) substrat(s) {Ai} et sur le produit cible {B}. Ces clones de phénotype (Ai+ ; B+) sont susceptibles de convertir {Ai} en {B} et sont donc soumis à l'étape suivante ;
   - type 2 : des clones recombinants capables de croître seulement sur un (ou plusieurs) substrat(s) {Ai} mais pas sur le produit cible {B}. Ces clones de phénotype (Ai+ ; B-) sont *a priori* incapables de produire le produit cible {B} ; et,
   - type 3 : des clones recombinants capables de croître seulement sur le produit cible {B}. Ces clones de phénotype (Ai- ; B+) peuvent avantageusement permettre le développement d'une souche receveuse de l'organisme hôte utilisable pour détecter par sélection directe tout clone recombinant capable de synthétiser le produit cible {B}. Ces clones peuvent être utilisés dans une réalisation alternative de transformation-sélection exposée ci-après.
      La capacité de croissance sur {Ai} et {B} peut être associée ou être indépendante (cf figure 2)
D- La mutagenèse *in vitro* des acides nucléiques issus de la banque de séquence transformante et isolés des clones présentant un phénotype (Ai+ ; B+).
E- La sélection parallèle sur {Ai} et sur {B} des clones résultant de la transformation par les acides nucléiques mutés, permet l'identification des clones transformés affectés par la mutagenèse (figure 3). Cette sélection permet de sélectionner :
   - des clones transformés issus de mutation, de phénotype (Ai- ; B-), ayant perdu la capacité de croître sur {Ai} et sur {B}. Ce changement de phénotype peut s'expliquer soit parce que (i) la voie métabolique de {Ai} passe par {B} et que le métabolisme de {B} est disrupté (phénotype muté IIIa), soit parce que (ii) la mutagenèse a atteint un élément commun à {Ai} et {B} comme par exemple un élément de régulation, un transporteur commun etc (phénotype IIIb).
   - des clones transformés issus de mutation, de phénotype (Ai- ; B+), ayant seulement perdu la capacité de croître sur {Ai}. La voie métabolique d'intérêt permettant la transformation de {Ai} en {B} est disruptée (phénotype muté IV).
F- L'analyse quantitative de {Ai} et {B} par des méthodes analytiques directes ou indirectes, à travers les phénotypes (Ai- ; B-).
G- La caractérisation génétique du biocatalyseur (fonction : {Ai} est converti en {B}) à travers les phénotypes (Ai- ; B+) ou les phénotypes (Ai- ; B-) accumulant le produit {B} sur un milieu riche supplémenté en {Ai}).

Seuls les phénotypes (Ai- ; B+) et (Ai- ; B-) des clones transformants issus de mutagenèse *in vitro* permettent l'identification et la caractérisation des nouvelles voies métaboliques recherchées transformant {Ai} en {B}. L'accumulation de {B} et sa détection chimique est réalisée par culture des phénotypes (Ai+ ; B+), ou préférentiellement (Ai- ; B-), sur un milieu riche supplémenté en {Ai}.

Le procédé de l'invention présente l'avantage de ne considérer dans ce type de sélection primaire que les clones positifs car seuls ces derniers ont la capacité de se développer. Ainsi, il n'est pas nécessaire de cribler tous les clones contenus dans la banque.

Dans une réalisation alternative de l'invention, lorsque de l'étape de sélection primaire du premier mode de réalisation ne permet pas de détecter de clones présentant un phénotype (Ai+ ; B+), les phénotypes (Ai- ; B+) éventuels fournissent la possibilité de développer une souche receveuse de phénotype (Ai- ; B+) susceptible d'être co-transformée par une seconde banque metagénomique. Cette réalisation alternative permet d'exploiter au sein de la banque metagénomique les clones (Ai+ ; B-) capables de convertir au moins un des substrats {Ai} en produit cible {B} mais incapables de métaboliser {B} (clones non sélectionnés dans l'étape de sélection primaire du premier mode de réalisation).

Basée sur un système de transformation-sélection, l'invention :
- permet de sélectionner directement des voies métaboliques convertissant un (ou plusieurs) substrat(s) parfaitement caractérisé(s) en un produit cible d'intérêt ;
- permet en parallèle de sélectionner directement des voies métaboliques métabolisant un (ou plusieurs) substrat(s) parfaitement caractérisé(s) comme source(s) unique(s) d'un élément essentiel. Après caractérisation génétique et chimique, ces voies métaboliques permettent d'enrichir des banques d'enzymes spécialisées ;
- permet de façon alternative de développer aisément une souche receveuse de l'organisme hôte, capable de croître sur un produit cible d'intérêt, et utilisable *de facto* pour un second cycle de transformation-sélection pour la conversion d'un ou plusieurs substrats parfaitement caractérisés en ce produit cible d'intérêt. Cette souche receveuse de l'organisme hôte est caractérisée en ce fait que son développement résulte de l'intégration temporaire d'un vecteur recombinant et que son patrimoine génétique propre est demeuré inchangé ;
- permet d'exploiter, lors de chaque cycle de transformation-sélection, l'énorme potentiel génétique de banques metagénomiques à très grands effectifs, sans qu'il soit nécessaire de structurer au préalable ces banques et, *de facto,* sans qu'il soit nécessaire d'avoir recours à des systèmes de criblage à haut débit.

L'absence de structuration au préalable des banques permet dès lors de déplacer les efforts sur la réalisation de banques metagénomiques optimisant les chances de découverte de la voie métabolique cible.

L'invention concerne en outre la sélection d'une cellule hôte incapable de métaboliser un substrat {Ai} et incapable de métaboliser un produit {B} recherché. De manière préférentielle, cette cellule hôte est un hôte bactérien. Il s'agit de manière non-limitative de *E. coli, Bacillus, Streptomyces, Pseudomonas, Nocardia, Acinetobacter* etc... Cette cellule doit posséder la capacité d'être transformable par l'une des techniques connues de l'homme du métier. Il peut s'agir de manière non limitative de transformation par électroporation, par conjugaison, par transduction, par infection, etc.... Cette cellule est utilisée comme hôte d'expression pour individualiser des fragments d'ADN vectorisés.

### Définitions

Metagénome désigne l'ensemble des génomes d'une communauté microbienne d'un environnement donné.

Metagénomique désigne, au sens strict du terme, l'analyse globale d'un metagénome indépendamment de toute culture artificielle des microorganismes. Dans l'acception commune, au-delà de l'étude directe de l'information génétique (ADN metagénomique), la metagénomique s'appuie sur la réalisation préalable de banques metagénomiques.

Banque d'ADN metagénomique désigne une population d'ADNs metagénomiques clonés dans un vecteur de clonage ou d'expression, permettant le transfert et le maintien de cet ADN metagénomique dans un (ou plusieurs) organisme(s) hôte(s). La banque metagénomique peut être non redondante, en ce fait que chaque molécule d'ADN métagénomique clonée est unique, ou peut être amplifiée, en ce fait que chaque molécule d'ADN métagénomique clonée a été multipliée.

Banque metagénomique désigne une population de clones d'un organisme hôte ayant incorporé la population d'ADNs metagénomiques clonés, précédemment citée (clones recombinants). La banque metagénomique peut être non redondante, en ce fait que chaque clone recombinant est unique, ou peut être amplifiée, en ce fait que chaque clone recombinant a été multiplié. L'amplification d'une banque non redondante originelle permet ainsi le fractionnement de la banque amplifiée en sous-banques, au sein desquelles la diversité demeure représentative à la fois de celle de la banque amplifiée et de celle de la banque non redondante originelle.

Vecteur recombinant désigne un vecteur d'expression ou de clonage ayant intégré une information génétique exogène, par exemple ADN génomique ou ADN metagénomique.

Clone recombinant désigne une population de cellules clonales identiques d'un organisme hôte ayant intégré un vecteur recombinant, par exemple par transformation génétique.

Voie biocatalytique désigne un ensemble de protéines catalytiques (enzymes) réalisant la conversion d'un composé de départ (substrat) en un composé final (produit cible).

Famille de voie métabolique désigne l'ensemble des séquences d'acides nucléiques dont le produit d'expression est capable de réaliser la transformation d'un substrat {A] en un produit {B}.

Vecteur navette désigne un vecteur permettant le transfert et le maintien d'une information génétique d'une (ou plusieurs) espèce(s) ou souche(s) bactérienne(s) donneuse(s) vers un ou plusieurs organisme(s) ou souche(s) ou espèce(s) hôte.

### Description des environnements

L'invention s'appuie tout d'abord sur la constitution de banques metagénomiques provenant d'un échantillon environnemental. Le sol et les sédiments constituent des environnements majeurs pour la recherche de nouveaux métabolites actifs, non seulement du fait de la très grande quantité de microorganismes qu'ils contiennent, mais aussi du fait de la diversité considérable de ces microorganismes. Les microorganismes ont été détectés dans un très grand nombre d'environnements, allant de la stratosphère aux profondeurs abyssales, en incluant des habitats extrêmes en terme de conditions physicochimiques qui y prévalent. L'invention s'applique de façon non limitative à des échantillons issus du sol, de sédiments, de milieux aquatiques (eau douce ou marine), de plantes, d'insectes, d'animaux, de bioréacteurs tels que les biofilms, fermenteurs et boues activées, mais aussi des environnements dérivés animaux ou humains (comme par exemple le rumen, les fécès), et avantageusement tous les environnements présentant un avantage quantitatif (forte concentration en microorganismes) ou qualitatif (spécificité de la ou des communautés microbiennes).

L'échantillon environnemental contient une multitude d'organismes incluant des eubactéries, des archaebactéries, des algues, des champignons, des levures, des protozoaires, des virus, des phages, des parasites, etc. Les microorganismes peuvent être représentés par des extrémophiles comme les thermophiles, psychrophiles, acidophiles, halophiles, etc. L'échantillon environnemental peut contenir des microorganismes cultivables ou non-cultivables, connus ou inconnus, ainsi que des acides nucléiques libres et de la matière organique.

### Préparation des acides nucléiques

L'ADN environnemental est récupéré à partir d'organismes cultivables ou non cultivables par l'une des techniques connues de l'homme du métier. Deux principales approches sont généralement mises en oeuvre pour l'extraction d'ADN environemental. La première approche, dite approche directe, consiste en l'extraction des acides nucléiques de l'échantillon à travers une lyse *in situ* des microorganismes, suivie d'une purification extensive des acides nucléiques libérés. La lyse des cellules bactériennes peut être effectuée par la mise en oeuvre simple ou combinée de multiples procédés de disruption physique, chimique et/ou enzymatique des parois et membranes cellulaires, procédés connus de l'homme de métier (pour revue, voir Robe et al., 2003, Eur. J. Soil Biol., 39 : 183-190). La purification extensive des acides nucléiques libérés lors de la lyse peut mettre en oeuvre, de manière individuelle ou combinée, de nombreuses méthodes connues de l'homme de métier, incluant de façon non limitative l'ultracentrifugation sur gradients de chlorure de cesium, le passage sur colonnes d'hydroxyapatite, l'electrophorèse sur gel d'agarose, la filtration sur résines, ou tout autre procédé commercialisé de purification d'acides nucléiques (pour revue, voir Robe *et al.,* 2003). La seconde approche, dite approche indirecte, consiste en une séparation préalable des microorganismes de l'échantillon, de façon non limitative, par centrifugations différentielles ou par centrifugations sur gradients de densité, suivie de la lyse des microorganismes ainsi séparés, puis de la purification extensive des acides nucléiques libérés. Les étapes de lyse des microorganismes et de purification des acides nucléiques sont communément effectuées par la mise en oeuvre, de manière individuelle ou combinée, de nombreuses méthodes connues de l'homme du métier et évoquées ci-avant.

L'efficacité comparée de ces deux approches ainsi que leurs avantages et inconvénients respectifs ont fait l'objet de nombreuses études scientifiques, et sont connus de l'homme du métier. La mise en place de la stratégie d'extraction des acides nucléiques, *i.e*. le choix de l'une ou l'autre de ces deux approches ainsi que le choix des différentes méthodes, reposent de façon non limitative sur les caractéristiques de l'environnement visé, sur les microorganismes ciblés (tout ou partie des microorganismes de cet environnement) et leurs caractéristiques, sur la taille des acides nucléiques, ainsi que sur le choix du vecteur de clonage et de la stratégie de clonage choisie.

### Organismes hôtes

Tout système hôte-vecteur connu dans l'état de l'art peut être utilisé dans la présente invention. L'identification d'une cellule hôte constitue l'Etape 1 de la présente invention. La cellule hôte peut être eucaryote ou procaryote. De manière préférentielle, la cellule hôte utilisée est un hôte bactérien. Il s'agit de manière non-limitative de *Escherichia coli, Bacillus subtilis, Streptomyces lividans, Pseudomonas, Nocardia, Acinetobacter* etc. Des exemples de cellules hôtes eucaryotes sont, sans être limités à ceux-ci, les levures et les champignons. La cellule hôte (i) peut provenir de collections de souches publiques, de laboratoires privés ou de sociétés commerciales ; (ii) doit être sélectionnée ou modifiée pour son incapacité à métaboliser le(s) substrat(s) {Ai} et le produit cible {B} ; (iii) doit pouvoir être cultivable dans les conditions standards connues de l'homme de l'art; (iv) doit posséder la capacité d'être transformable par l'une des techniques connues de l'homme de l'art ; (v) doit enfin maintenir de façon stable les ADN exogènes transformants malgré d'éventuels systèmes comme la recombinaison, la restriction, etc...

### Vecteurs de clonage et d'expression

Les acides nucléiques sont clonés à l'intérieur d'un vecteur d'expression approprié, le maintien de l'ADN étant réplicatif. Le vecteur d'expression utilisé dépend de la taille des acides nucléiques purifiés, de la taille d'insert souhaitée *in fine* (communément comprise entre 5 kb et plus de 100 kb), et de l'hôte d'expression choisi qui est de manière préférentielle un hôte bactérien.

De nombreux vecteurs de clonage ou d'expression ont été décrits dans l'art antérieur. Il s'agit de manière non limitative de plasmides, cosmides tels que ceux commercialiés par les Sociétés Stratagene (SuperCos et pWE15) et Epicentre Technologies (pWeb cosmide cloning kit), fosmides tels que décrits par Kim et al. (1992, Nucl. Acids Res. 20 : 1083-1085), chromosomes artificiels PAC comme décrits par Ioannou et al., (xxx Nat. Genet., 6 : 84-89), chromosomes artificiels BAC comme décrits par Shizuya et al. (1992, Proc. Natl. Acad. Sci., 89:8794-8797), chromosomes artificiels YAC comme décrits par Larin et al. (1991, Proc. Natl. Acad. Sci., 88 : 4123-4132), phagemides et vecteurs dérivés de phages tels que ceux commercialisé par la Société Stratagene (Lambda Dash II et Zap II), vecteurs viraux, etc. Préferentiellement, les vecteurs sont de type cosmide, fosmide, BAC, YAC et P1-dérivés car ils permettent le clonage de fragments d'ADN de grande taille (de 30 kb à 200 kb et plus pour les BACs et les YACs). Les vecteurs peuvent être soit intégratifs, en ce fait qu'ils s'intègrent aléatoirement ou de façon dirigée dans le génome de la cellule hôte, soit préférentiellement réplicatif, en ce fait que le vecteur se maintien dans la cellule hôte indépendemment du génôme de cette cellule. Par définition les vecteurs de clonage contiennent un certain nombre d'éléments nécessaires au maintien du vecteur dans la cellule hôte (origine de réplication fonctionnelle), ou encore nécessaires à la sélection et/ou la détection du vecteur dans cette cellule (gène marqueur comme par exemple un gène de résistance à un antibiotique sous promoteur fonctionnel dans la cellule hôte et permettant une pression de sélection positive). Du fait de la spécificité de ces éléments constitutifs, les vecteurs présentent un spectre d'hôte plus ou moins large.

### Clonage

La réalisation du clonage, *i.e*. l'introduction des séquences d'acide nucléique, de préférence d'ADNs metagénomiques purifiés, dans le vecteur approprié, fait appel à de multiples étapes de manipulation moléculaire des ADNs (de façon non limitative des restrictions, déphosphorylations, ligations, etc.) qui ont été largement décrites, par exemple dans Current Protocols in Molecular Biology, Eds. F.M. Ausubel, R. Brent, R.E. Kingston, D.D. Moore, J.G. Seidman, J.A. Smith and K. Struhl, publié par Greene Publishing Associates and Wiley Inter-Science. Deux approches de réalisation des banques metagénomiques sont envisageables.

Dans un premier mode de réalisation préférentiel, la construction de la banque metagénomique est directement réalisée dans un vecteur navette spécifique d'un ou plusieurs hôtes, de préférence bactériens, par exemple comme décrit dans les brevets N° WO 01/40497A2 (Aventis Pharma, 1999) et WO 99/67374 (Biosearch Italia, 1999) pour *Streptomyces.* Dans un second mode de réalisation, les acides nucléiques purifiés sont clonés dans un vecteur généraliste, par exemple de type fosmide ou BAC, puis les vecteurs recombinants sont modifiés, individuellement ou en pool, avantageusement par transposition comme décrit dans la demande de brevet N° PCT/EP 03/07765 (Libragen). Dans cette démarche, la transposition permet d'introduire, soit dans le vecteur, soit dans l'insert (disruption ou activation), les éléments génétiques nécessaires au transfert, à la réplication ou à l'intégration du vecteur recombinant chez la cellule hôte choisie, de préférence un hôte bactérien. Cette post-modification des clones de la banque peut être effectuée individuellement (banque metagénomique structurée en format de microplaques 96 ou 384) ou collectivement (banque metagénomique non structurée). La transformation de la population de cellules hôtes identifiée à l'étape 1) par une population d'ADNs clonés constitue **l'Etape 2** de la présente invention. Dans les deux modes de réalisation, la banque metagénomique peut être préalablement structurée, en ceci que l'ensemble des clones de la banque sont individualisés dans un format susceptible d'automatisation (microplaques 96, 384, 1536) ou être préférentiellement conservée sous forme d'un mélange de clones recombinants. Dans ce mode préférentiel de conservation, la banque peut être avantageusement amplifiée, en ceci que les cellules hôtes, après transformation ou infection, sont multipliées pendant un nombre de cycles déterminé, conduisant à ce que chaque clone recombinant de la banque soit représenté en n copies dans la banque amplifiée, et à ce que la banque amplifiée puisse faire l'objet de multiples tests de criblage ou de sélection simultanés, sans perte de diversité.

### Détection et identification des voies métaboliques

**Etape 3** : Les clones recombinants sont directement sélectionnés, sans étape de culture préalable, sur un milieu de culture minimum contenant à la fois n substrats Ai (i de 1 à n) et le produit cible {B} comme seules sources d'un élément essentiel, ainsi qu'un antibiotique (comme le chloramphenicol) permettant de maintenir une pression de sélection sur les cellules hôte ayant intégré un vecteur recombinant. Cette étape de sélection primaire, portant sur une banque originale ou amplifiée de quelques dizaines voir plusieurs centaines de milliers de clones, permet de ne *considérer in fine* que les seuls clones recombinants capables de métaboliser l'un des substrats {Ai} indépendamment du produit cible {B}, ou le produit cible {B}, ou l'un des substrats {Ai} à travers {B}. Cette étape est toutefois optionelle.

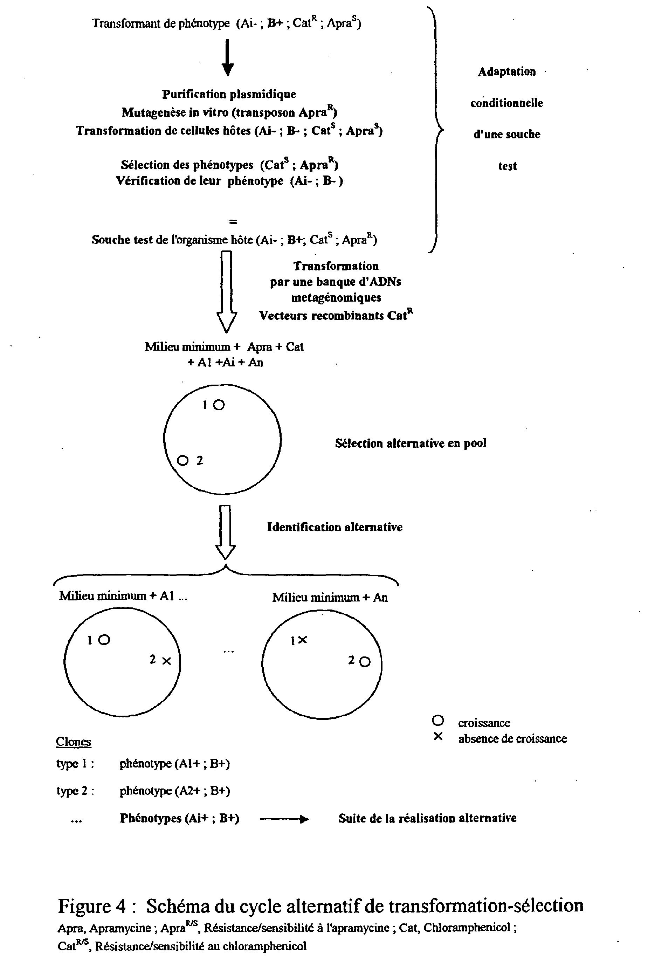

**Etape 4** : Les clones sélectionnés sur milieu minimum contenant les n substrats {Ai} et {B} (figure 2, clones 1, 2 et 3), sont conservés puis testés en parallèle sur milieu minimum contenant soit l'un des substrats {Ai}, soit {B}. Pour un substrat donné {Ai}, cette sélection multiple permet d'identifier trois phénotypes distincts (figure 1) :
- le phénotype (Ai+ ; B+) des clones (de type 1). Il correspond au phénotype recherché préférentiellement correspondant à des clones capables de croître sur milieu minimum avec le substrat {Ai} mais aussi avec le produit cible {B} comme seule source d'un élément essentiel. Les clones présentant ce phénotype sont conservés et soumis à l'étape suivante (figure 3).
- le phénotype (Ai+ ; B-) des clones (de type 2). Il correspond à des clones capables de croître sur milieu minimum avec le substrat {Ai} comme seule source d'un élément essentiel indépendamment du produit cible {B}. S'ils ne sont pas traités lors des étapes suivantes, ces clones sont néanmoins conservés car susceptibles d'enrichir des banques d'enzymes après identification des voies métaboliques impliquées dans la métabolisation du substrat {Ai}.
- le phénotype (Ai- ; B+) des clones (de type 3). Il correspond à des clones incapables de croître sur milieu minimum avec le substrat {Ai} comme seule source d'un élément essentiel mais qui sont en revanche capables de métaboliser le produit cible {B} pour croître. Ces clones peuvent avantageusement faire l'objet du développement d'une souche receveuse de l'organisme, présentant un phénotype (Ai- ; B+), utilisable dans une réalisation alternative de transformation-sélection secondaire de la biocatalyse de {Ai} en {B} (figure 4). Ces clones sont également susceptibles d'enrichir des banques d'enzymes après identification des voies métaboliques impliquées dans la métabolisation du produit {B}.

**Etape 5** : L'ADN plasmidique des clones recombinants de phénotype (Ai+ ; B+), sélectionnés à l'étape 4 et capables de croître à la fois sur {Ai} et sur {B} est extrait par une des techniques connues de l'homme du métier (Sambrook *et al.,* 1989 (figure 3). Cet ADN plasmique est soumis à une disruption génétique, avantageusement par insertion aléatoire d'éléments transposables comme ceux commercialisés (EZ ::TN société Epicentre Technologies) et réintroduit dans l'organisme hôte (figure 3A). Les transformants sont d'abord étalés sur milieu solide riche jusqu'à l'apparition des colonies, puis les colonies sont transférées par répliques sur milieu minimum contenant seulement le substrat {Ai} d'une part, et seulement le produit cible {B} d'autre part. La capacité de croissance sur {Ai} et {B} peut être la résultat d'une voie métabolique commune (figure 3B, génotype [AB]) ou de deux voies métaboliques indépendantes (figure 3B, génotype [A,B]).

**Etape 6 :** La sélection parallèle sur {Ai} et {B} des clones issus de mutagenèse permet d'identifier différents phénotypes d'intérêt :
- le phénotype muté I (Ai+ ; B+)* de clones transposés capables de pousser sur {Ai} et {B} ; soit la mutagenèse par transposition n'a pas d'effet dans l'insert metagénomique ou soit elle porte sur le vecteur.
- le phénotype muté II (Ai+ ; B-)* de clones transposés capables de métaboliser {Ai} pour croître mais pas {B}. Le métabolisme de {Ai} ne passe pas par la production de {B} pour permettre la croissance (figure 2, génotype [A,B]).
- le phénotype muté III (Ai- ; B-)* de clones transposés capables de n'utiliser ni {Ai} ni {B} comme sources de croissance. Soit (i) la mutagenèse par transposition a atteint un élément commun aux voies métaboliques de {Ai} et {B} comme par exemple un élément de régulation, un transporteur commun, etc ; soit (ii) la voie métabolique de {Ai} passe par {B} et la voie métabolique de {B} permettant la croissance est disruptée.
- le phénotype muté IV (Ai- ; B+)* de clones transposés capables d'utiliser {B} mais pas {Ai} pour croître. La voie métabolique d'intérêt permettant la conversion du substrat {Ai} en le produit cible {B} est disruptée.

**Etape 7 :** La vérification du passage par {B} dans la métabolisation de {Ai} se fait par évaluation de l'accumulation de {B} par des techniques de chimie analytique quand un clone de phénotype (Ai- ; B-), isolé à l'étape 4, se développe sur milieu riche.

**Etape 8 :** La caractérisation génétique du biocatalyseur, *i.e*. caractérisation du ou des gène(s) codant pour l'enzyme ou les enzymes impliquées dans la conversion de {Ai} en {B}, est réalisée grâce aux clones transposés présentant le phénotype (Ai-B+). L'analyse génétique des séquences nucléiques situées au niveau du ou des site(s) de disruption des clones recombinants (Ai- ; B+) permet d'élucider le(s) système(s) génétique(s) responsable(s) de la conversion de {Ai} en {B}. L'analyse génétique est réalisée par toutes méthodes connues de l'homme de métier, incluant de façon non limitative la détermination des séquences d'acides nucléiques, l'identification des séquences codantes et régulatrices, etc. Cette méthode permet d'accéder rapidement et directement (une seule étape) à une famille de voie métabolique capable de transformer un substrat {Ai} en un produit {B}.

### Réalisation alternative de transformation-sélection

Dans une réalisation alternative de l'invention, notamment lorsque de l'étape 4 du premier mode de réalisation ne permet pas de détecter de clones présentant un phénotype (Ai+ ; B+), les phénotypes (Ai- ; B+) éventuels fournissent la possibilité de développer une souche receveuse de phénotype (Ai- ; B+) susceptible d'être co-transformée par une seconde banque metagénomique (figure 4). Cette banque peut être la même que la première banque ou peut être une banque distincte. Cette réalisation alternative permet d'exploiter au sein de la banque metagénomique les clones capables de convertir au moins un des substrats {Ai} en produit cible {B} mais incapables de métaboliser {B} (clones non sélectionnés dans l'étape 4 du premier mode de réalisation). Ce mode de réalisation alternative procède de plusieurs étapes successives :
a) Fournir une population de cellules hôtes transformables (Ai- ; B+). Cette étape, facultative, n'est nécessaire que si l'on souhaite transformer la population de cellules hôtes (Ai- ; B+) par une banque d'ADN metagénomique ou génomique sélectionable par le même marqueur de résistance. Cette étape facultative procède de plusieurs étapes successives (Figure 4) :
   - purification plasmidique d'un ou plusieurs clones présentant le phénotype (Ai- ; B+),
   - mutagenèse *in vitro,* avantageusement par transposition comme précédemment décrit précédemment à l'étape 5 avec un élément transposable portant une résistance fonctionnelle à un antibiotique absente sur le vecteur cible (par exemple résistance à l'apramycine), sur le ou les clones purifiés,
   - transformation d'une population de cellules hôtes (Ai- ; B-) et sélection des mutations affectant le gène de résistance à l'antibiotique du vecteur cible (par exemple résistance au chloramphenicol). La vérification de la croissance de cette souche receveuse sur milieu minimum avec le produit cible {B} comme seule source de carbone permet d'exclure qu'un autre événement de mutation (transposition) ait pu altérer la fonction d'utilisation de {B} pour la croissance. La souche receveuse de l'organisme est alors disponible, capable de croître sur apramycine et sur milieu minimum contenant le produit cible {B} comme seule source d'un élément essentiel.

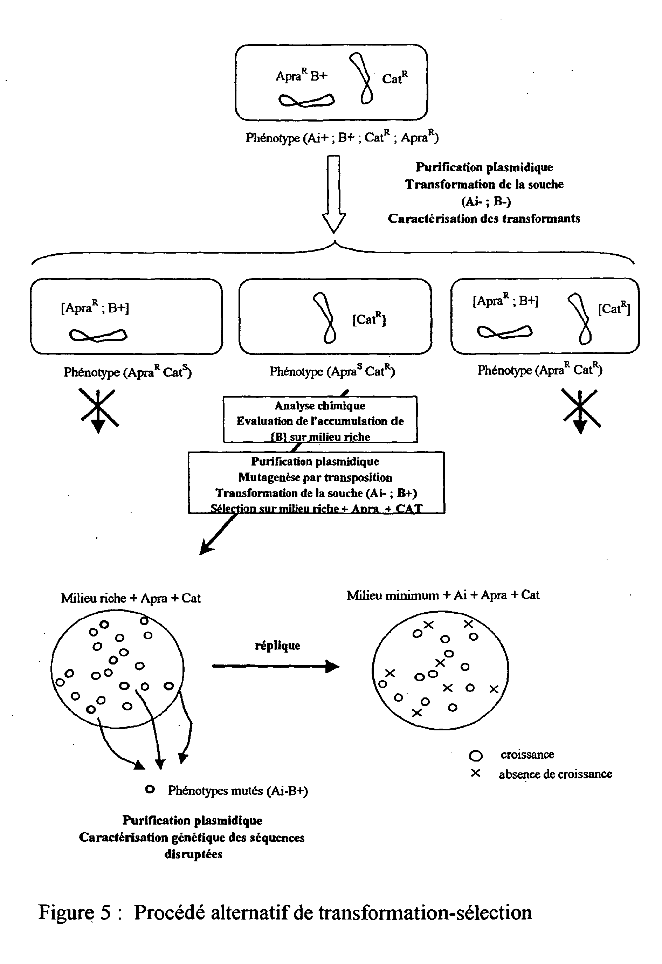

- b) Transformation de la souche receveuse par une banque d'ADN metagénomique et sélection de clones recombinants sur milieu minimum contenant n substrats {Ai} (i de 1 à n) comme seule(s) source(s) d'un élément essentiel et les deux antibiotiques de la souche receveuse et des vecteurs recombinants.
- c) Les clones sélectionnés sur milieu minimum contenant les n substrats {Ai} (figure 4, clones 1, 2), sont conservés puis testés en parallèle sur milieu minimum contenant l'un des substrats {Ai}. Pour un substrat donné {Ai}, cette sélection multiple permet d'identifier des colonies présentant un phénotype (Ai+ ; B+) traduisant *a priori* la capacité du clone à croître sur le produit cible {B} à travers la conversion du substrat {Ai} en {B}.
- d) Purification plasmidique d'un ou plusieurs clone(s) présentant le phénotype (Ai+ ; B+). L'échantillon de plasmides purifiés (figure 5) contient en mélange le vecteur recombinant Apra^{R} (voie métabolique permettant la croissance sur le produit cible {B}) et le vecteur recombinant Cat^{R} (voie métabolique réalisant la conversion du substrat {Ai} en {B}).
- e) La cellule hôte de phénotype (Ai- ; B-) est transformée avec le mélange de vecteurs recombinants purifiés en e) et les clones recombinants Cat^{R} Apra^{S} sont sélectionnés. La vérification de la conversion du substrat {Ai} en {B} se fait par évaluation de l'accumulation de {B} par des techniques de chimie analytique quand ces clones Cat^{R} Apra^{S} se développent sur milieu riche. L'accumulation de {B} confirme que ces clones Cat^{R} Apra^{S} présentent bien un phénotype (Ai+ ; B-).
- f) Purification plasmidique d'un ou plusieurs clone(s) présentant le phénotype (Cat^{R} Apra^{S}) identifiés en f). Le vecteur recombinant est soumis à une disruption génétique, avantageusement par insertion aléatoire d'éléments transposables.
- g) Transformation de la souche receveuse (Ai- ; B+) par la population de vecteurs recombinants mutagénisés en g). Les transformants sont d'abord étalés sur milieu solide riche jusqu'à l'apparition des colonies, puis les colonies sont transférées par répliques sur milieu minimum contenant seulement le substrat {Ai} comme source d'un élément essentiel. La sélection parallèle des clones issus de mutagenèse sur milieu riche et sur milieu minimum contenant seulement le substrat {Ai} permet d'identifier les clones devenus incapables de croître avec le substrat {Ai} comme source d'un élément essentiel, *i.e*. chez lesquel la disruption affecte la voie métabolique convertissant {Ai} en {B}.
- h) La caractérisation génétique du biocatalyseur, *i.e*. caractérisation du ou des gène (s) codant pour l'enzyme ou les enzymes impliquées dans la conversion de {Ai} en {B}, est réalisée gràce aux clones transposés présentant le phénotype (Ai- ; B+). L'analyse génétique des séquences nucléiques situées au niveau du ou des site(s) de disruption des clones recombinants (Ai- ; B+) permet d'élucider le(s) systèmes génétiques responsables de la conversion de {Ai} en {B}.

### Exemples

### Exemple 1: Recherche de la voie métabolique de bioconversion de phytostérols en 4-androstene-3,17-dione (AD)

Le 4-androstene-3,17-dione (AD, CAS N° 63-05-8) et le 1,4-androstadiene-3,17-dione (ADD, CAS N°897-06-3) constituent d'importants intermédiaires pour l'industrie pharmaceutique, comme précursseurs clés de la production de stéroides thérapeuthiques. De nombreux microorganismes possèdent la capacité naturelle de dégrader des 3β-hydroxy-Δ5-stérols (par exemple du β-sitostérol, du campestérol ou du brassicastérol) en formant de l'AD et de l'ADD comme intermédiaires de dégradation.

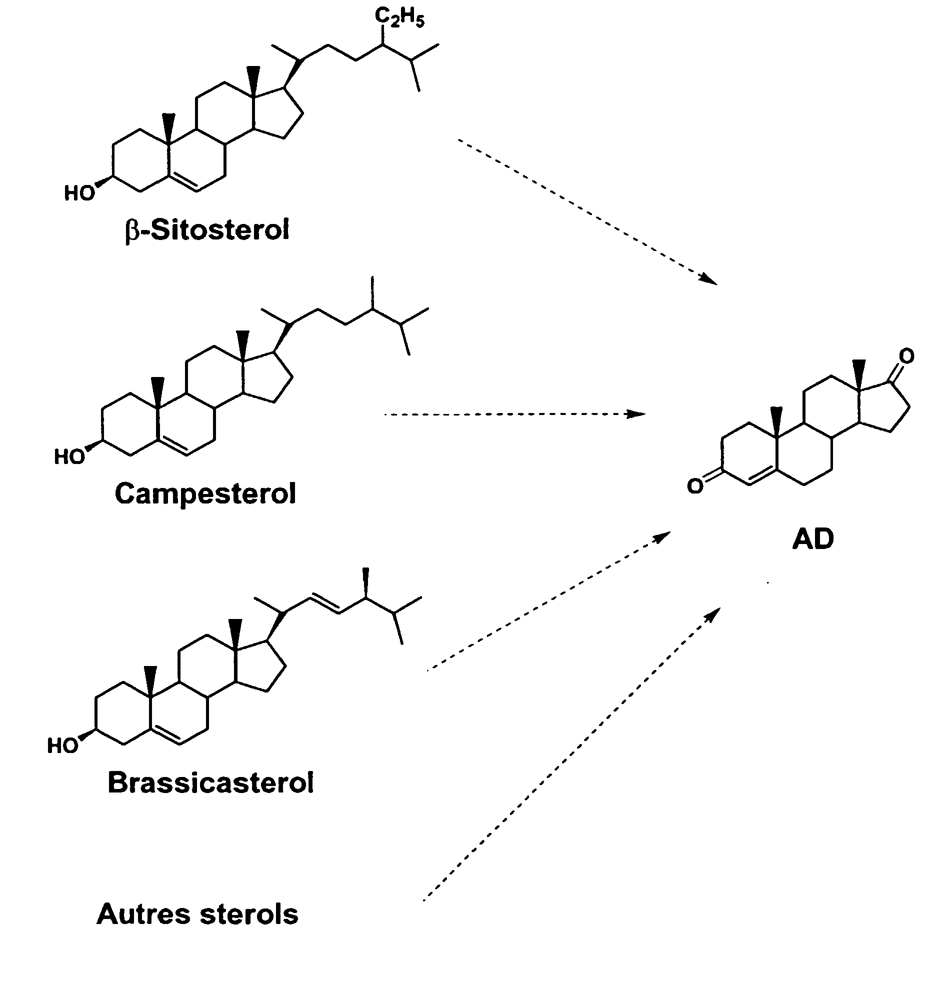

La conversion microbienne de phytostérols naturels en AD par des souches bactériennes caractérisées a été largement décrite (Shashabi B. Mahato et al, 1997 Advances in microbial steroid biotransformation, steroids, 62, 332-345), notamment par des souches mutantes *de Mycobacterium* sp. Cette bioconversion se heurte cependant à un certain nombre de contraintes : la faible solubilité des phytostérols utilisés comme substrats, les faibles rendements associés à des temps importants de fermentation ainsi que la production concomitante d'AD et d'ADD qui nécessite une séparation difficile et couteuse de ces deux produits stéroidiens.

Par ailleurs, des études scientifiques (van der Geize et al., 2002, Microbiology, 148 :3285-3292) conduites sur *Rhodococcus erythropolis* ont démontré que l'inactivation de l'enzyme 3-ketosteroid dehydrogenase (KSTD), impliquée dans le catabolisme de l'AD, ne suffisait pas à empécher la croissance de *R. erythropolis* sur AD, comme seule source de carbone et d'énergie.

La stratégie mise en oeuvre vise à détecter les voies métaboliques permettant la conversion spécifique de phytostérols d'origine différente en AD en s'affranchissant des voies cataboliques de l'AD. Les voies métaboliques sont prospectées en parallèle au sein d'une banque génomique BAC de *Mycobacterium vaccae* et au sein d'une banque BAC d'ADNs metagénomiques issus de sol.

L'organisme hôte retenu est *Streptomyces lividans,* qui répond à l'ensemble des critères : bactérie cultivable, transformable, susceptible d'exprimer des voies métaboliques issus de *Mycobacterium* et des bactéries à haut taux GC du sol, connues pour leur capacité de dégradation des phytostérols, et incapable de croître sur milieu minimum supplémenté en phytostérols et androstenedione comme seules sources de carbone et d'énergie.

*S. lividans* est transformée par les banques d'ADN précitées. Les transformants sont déposés sur un milieu solide M9 additionné de 10µg/L de chloramphénicol contenant 0,5% de phytostérols et de 0,5% d'androstenedione (AD) comme unique source de carbone et d'énergie. Ils sont incubés 5-10 jours à 30°C. Les clones retenus capables de croître sont ensuite testés séparément sur milieu minimum supplémenté en phytostérols d'une part et en AD d'autre part. La capacité de croître sur phytostérols et/ou sur AD est liée aux ADNs introduits lors de la transformation.

Les clones capables de croître à la fois sur phytostérols et sur AD comme seules sources de carbone sont sélectionnés, leurs vecteurs sont ré-extraits puis soumis à mutagenèse in vitro par transposition (EZ :TN Epicentre Technologies transposition kit). Les vecteurs recombinants mutés sont réintroduits chez S. lividans. Les transformants sont déposés sur milieu riche et sont testés en parallèle sur un milieu solide M9 additionné de 10µg/L de chloramphénicol contenant 0,5% de phytostérols d'une part, et de 0,5% d'androstenedione (AD) d'autre part, comme unique source de carbone et d'énergie. Les clones de *S. lividans* ayant perdu la capacité de croître sur phytostérols mais capables de croître sur AD sont sélectionnés, et leur vecteur recombinant ré-extrait. La caractérisation génétique de voies métaboliques impliquées dans la conversion des phytostérols en AD est réalisée par séquençage desdits vecteurs recombinants sélectionnés ci-avant.

### Exemple 2: Recherche de la voie métabolique de bioconversion du 1-phényl-2-propanone en 1-phényl-2-propanol

Le phényl-2-propanol (CAS N° 103-79-7) est un composé largement utilisé comme motif structural composant de nombreux principes actifs (Liese, A. et al, 2000, Industrial Biotransformation ed Wiley-VCH 103-106). Il est retrouvé notamment comme intermédiaire pour la synthèse des amphétamines (Bracher, F. et al, 1994, Arch. Pharm. 327, 591-593). Des bactéries comme *Rhodococcus erythropolis* (Liese, A. et al 2000), mais aussi la levure *Saccharomyces Cerevisiae* (Gillois, J. et al, 1989, Journal of Organometallic Chemistry 367(1-2), 85-93) ont été décrites pour catalyser la réaction A présentée ci-dessous mais avec des rendements réactionnels de l'ordre de 70%.

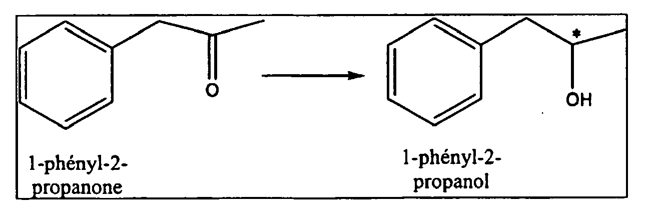

### Réaction A : Bioconversion du 1-phényl-2-propanone en 1-phényl-2-propanol.

La stratégie suivante est mise en oeuvre pour sélectionner une nouvelle et très efficace famille de voies métaboliques qui catalyse la bioconversion du 1-phényl-2-propanone en 1-phényl-2-propanol (CAS N° 698-87-3).

Les milieux de culture sont stérilisés à l'autoclave à 121 °C pendant 20 minutes. Le Casamino acide, le L-Tryptophane et la Thiamine HCl sont stérilisés à froid à travers une membrane millipore 0,2 µm et sont ajoutés au milieu de culture après stérilisation. Le 1-phényl-2-propanone et le 1-phényl-2-propanol sont dissous dans de l'éthanol et stérilisés à froid puis filtrés à travers une membrane millipore 0,2 µm avant d'être incorporés dans la gélose.

La souche hôte *E*. *Coli* DH10B (LifeTechnologies, Gibco BRL) est préalablement étalée sur un milieu solide M9 (composition pour 1 litre : 6.0 g de Na₂HPO₄ ; 3.0 g de KH₂PO₄; 1.0 g de NaCl ; 2.0 g de glucose ; 0.25 g de MgSO₄, 7H₂O; 15.0 mg de CaCl₂, 2H₂O ; 5.0 g de Casamino acides; 40.0 mg de L-Tryptophane ; 1.0 mg de Thiamine HCl ; qsp eau distillée) contenant 0,5% (V/V) de 1-phényl-2-propanone (Aldrich ref 13,538-0, CAS 103-79-7) ou de 1-phényl-2-propanol (Aldrich ref 14,923-5, CAS 14898-87-4) comme unique source de carbone et d'énergie. Les boîtes de Petri sont mises à incuber 18h-24h à 30°C. Aucun clone ne doit apparaître dans ces conditions, démontrant que la souche *E. Coli* DH10B est incapable de cataboliser le 1-phényl-2-propanone ou de 1-phényl-2-propanol.

Cette souche est ensuite transformée par les banques d'ADN environnementales préparées et réalisées selon les conditions opératoires décrites dans les brevets WO 01/81367 et EP N°02291871.8. Les transformants sont déposés sur un milieu solide M9 additionné de 10µg/L de chloramphénicol (stérilisé à froid, tel que décrit précédemment) contenant 0,5% de 1-phényl-2-propanone ou de 1-phényl-2-propanol comme unique source de carbone et d'énergie. Ils sont incubés 18h-24h à 30°C. Les clones retenus sont ceux qui sont capables de pousser avec comme unique source de carbone et d'énergie le 1-phényl-2-propanone et le 1-phényl-2-propanol. La possibilité d'utiliser ces 2 composés comme unique source de carbone et d'énergie est liée aux ADN métagénomiques portés par les vecteurs de clonage.

Les vecteurs qui portent les ADNs métagénomiques d'intérêt sont récupérés puis soumis à une mutagenèse aléatoire par exemple par insertion (kit transposon Epicentre Technologies EZ : TN). Ils sont ensuite réintroduits dans la souche *E. Coli* DH10B et les cellules ainsi transformées sont à nouveau déposées sur un milieu solide M9 additionné de 10µg/L de chloramphénicol et contenant 0,5% de 1-phényl-2-propanone ou de 1-phényl-2-propanol comme unique source de carbone et d'énergie. Ils sont incubés 18h-24h à 30°C.

Les clones retenus sont ceux qui se révèlent incapables de pousser avec le 1-phényl-2-propanol et le 1-phényl-2-propanone comme unique source de carbone. En effet, la voie de bioconversion du 1-phényl-2-propanone en 1-phényl-2-propanol est présente dans ces clones, et la mutation a inactivé cette voie. Une analyse chromatographique par Chromatographie liquide ou gazeuse permet à la fois de vérifier cette transformation et d'exclure d'éventuels faux positifs.

### Exemple 3 : Recherche de la voie métabolique de bioconversion du mandélonitrile en acide mandélique et du mandélonitrile en mandélamide

L'étude de l'hydrolyse microbiologique des nitriles en acide carboxylique ou en amide a été intensivement décrite (Wieser, M. et al (2000), Stereoselective biocatalysis Ed Patel RN "Stereoselective nitrile-converting enzymes" ; Ryuno, K. et al (2003), Yuki Gosei Kagaku Kyokaishi 61(5), 517-522 ; Okumura, M. (1991), JETI 39(6), 90-2 ; Endo, R., et al (2001), Jpn. Kokai Tokkyo Koho , Application: JP 2000-124591 20000425).

Le brevet EP-A-0 348 901 décrit la préparation d'acides R(-)-mandélique par hydrolyse du mandélonitrile racémique par une préparation soit *d'Alcaligenes fecalis,* souche ATCC 8750, soit de *Pseudomonas vesicularis,* souche ATCC 11426, soit de *Candida tropicalis,* souche ATCC 20311. Il propose de produire des acides carboxyliques α-substitués optiquement actifs à partir de nitriles ou d'amides α-substitués racémiques à l'aide de certains microorganismes du groupe constitué par les genres *Alcaligenes, Pseudomonas, Rhodopseudomonas, Corynebacterium, Acinetobacter, Bacillus, Mycobacterium, Rhodococcus,* ainsi que par une levure, à savoir *Candida.*

Dans le brevet EP-A-449 648 ou US-A-5 296 373, on décrit un procédé de production de l'énantiomère acide, R(-)-mandélique substitué à partir d'un mandélonitrile substitué racémique par mélange avec une préparation d'une bactérie *Rhodococcus,* souche HT 29-7 (FERM BP-3857) qui assure l'hydrolyse stéréosélective du groupe nitrile du racémique afin, apparemment, d'éviter les inconvénients de la séparation d'autres substances optiquement actives obtenues après hydrolyse par les microorganismes proposés dans EP-A-0 348 901. Le brevet EP-A-0 610 048 propose d'utiliser, dans une réaction similaire, des microorganismes du genre *Gordona,* tels que *Gordona terrae* MA-1 (FERM BP-4535).

Un des problèmes majeurs rencontrés dans ces réactions est l'apparition de nombreux sous produits (des aldéhydes) et le temps de demi-vie court de l'enzyme du fait de l'empoisonnement du catalyseur par les nitriles. L'objet du présent exemple propose de produire à partir de mandélonitrile soit l'acide mandélique chiral (Réaction B), soit le mandélamide (Réaction C) sans les problèmes généralement rencontrés et avec une forte activité spécifique et un rendement réactionnel important.

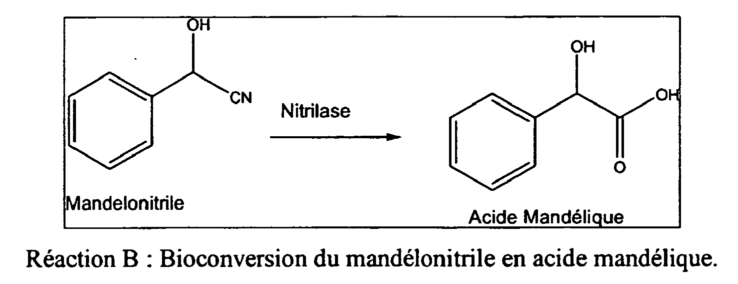

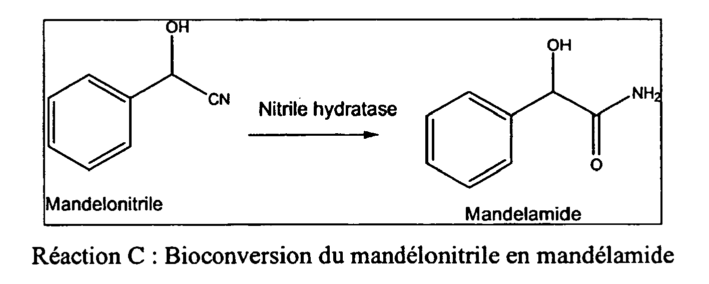

La stratégie utilisée est identique à celle mise en oeuvre dans l'exemple 1, avec simplement substitution des substrats de départ et des produits d'arrivée. Le mandélonitrile (CAS N°532-28-5), l'acide mandélique (CAS N°90-64-2) et le mandélamide (CAS N°4410-31-5) sont mis en solution dans de l'acétonitrile et préparés extémporanément avant leurs utilisations.

La confirmation des voies métaboliques catalysant la réaction recherchée est réalisée par une analyse en chromatographie liquide.

## Revendications

1. Méthode de sélection ou de préparation de cellules comprenant au moins une voie métabolique ou une famille de voies métaboliques permettant la transformation d'un ou plusieurs substrat(s) {Ai} en un produit recherche {B}, comprenant les étapes suivantes :
a) fournir une population de cellules hôtes (Ai- ; B-) incapables de métaboliser ledit ou lesdits substrats {Ai} et ledit produit {B} ;
b) transformer ladite population de cellules hôtes avec une banque de séquences d'acide nucléique ;
c) tester en parallèle ladite population de cellules hôtes transformées sur des milieux minimum contenant soit un des substrat(s) {Ai}, soit ledit produit {B} comme seule source d'un élément essentiel à la croissance; et,
d) sélectionner ladite ou lesdites cellule(s) höte(s) capable(s) de croissance milieu minimum contenant un des substrat(s) {Ai} et sur un milieu minimum contenant ledit produit {B} {Ai+; B+).

2. Méthode selon la revendication 1, comprenant, avant l'étape c), une étape consistant à tester population de cellules hôtes transformées sur un milieu minimum contenant le(s) substrat(s) {Ai} et ledit produit {B} comme seule source d'un élément essentiel à la croissance et sélectionner ladite ou lesdites cellule(s) hôte(s) capable(s) de croissance sur ledit milieu minimum contenant le(s) substrat(s) {Ai} et ledit produit {B}; ladite ou lesdites cellule(s) hôte(s) sélectionnée(s) étant ensuite soumise à l'étape c) et suivantes.

3. Méthode selon la revendication 1 ou 2, comprenant, après l'étape d), les étapes suivantes :
e) effectuer une mutagenèse *in vitro* de la molécule d'acide nucléique isolée de ladite ou lesdites cellule(s) hôte(s) transformées (Ai+ ; B+) à l'étape d) ;
f) re-transformer la population de cellules hôte (Ai- ; B-) décrite à l'étape a) avec la population des acides nucléiques mutés *in vitro* à l'étape e) et tester les cellule(s) hôte(s) ainsi transformées sur des milieux minimum contenant soit un des substrat(s) {Ai}, soit ledit produit {B} comme seule source d'un élément essentiel à la croissance; et,
g) sélectionner ladite ou lesdites cellule(s) hôte(s) transformées incapable(s) de croissance sur un milieu minimum contenant un des substrat(s) {Ai} et capable(s) de croissance sur un milieu minimum contenant ledit produit {B} (Ai- ; B+) puis facultativement isoler la molécule d'acide nucléique mutée.

4. Méthode selon la revendication 3, comprenant la caractérisation du ou des gènes codant pour l'enzyme ou les enzymes impliqué(s) dans la conversion du substrat {Ai} en produit {B} dans ladite ou lesdites cellule(s) hôte(s) (Ai- ; B+) sélectionnée(s) à l'étape g).

5. Méthode selon la revendication 3, comprenant, après l'étape f), en parallèle de l'étape g):
h) sélectionner ladite ou lesdites cellule(s) hôte(s) transformée(s) et devenue(s) incapable(s) de croissance sur un milieu minimum contenant un des substrat(s) {Ai} et sur un milieu minimum contenant ledit produit {B} (Ai- ; B.-) ;
i) effectuer une analyse quantitative de l'accumulation du produit {B} de ladite ou lesdites cellule(s) hôte(s) transformée(s) (Ai- ; B-) sur un milieu riche supplémenté en {Ai} ; et
j) sélectionner ladite ou lesdites cellule(s) hôte(s) transformées (Ai- ; B-) accumulant le produit {B} sur un milieu riche et facultativement isoler en parallèle la molécule d'acide nucléique mutée introduite lors de la transformation de l'étape f).

6. Méthode selon la revendication 5, comprenant la caractérisation du ou des gènes codant pour l'enzyme ou les enzymes impliquée) dans la conversion du substrat {Ai} en produit {B} dans ladite ou lesdites cellule(s) hôte(s) (Ai- ; B-) sélectionnée(s) à l'étape j).

7. Méthode selon l'une quelconque des revendications précédentes, comprenant, après l'étape c), en parallèle de l'étape d) et suivantes, les étapes suivantes :
k) sélectionner ladite ou lesdites cellule(s) hôte(s) transformée(s), incapable(s) de croissance sur un milieu minimum contenant un des substrat(s) {Ai} et capable(s) de croissance sur un milieu minimum contenant ledit produit {B}, appelée(s) cellule(s) receveuse(s)(Ai- ; B+) ;
l) transformer ladite ou lesdites cellule(s) receveuse(s) (Ai- ; B+) avec une banque de séquences d'acide nucléique;
m) tester en parallèle ladite ou lesdites cellule(s) receveuse(s) transformée(s) (Ai- ; B+) sur un milieu minimum contenant un des substrat(s) {Ai} ; et
n) sélectionner ladite ou lesdites cellule(s) receveuse(s) transformée(s) capable(s) de croissance sur un milieu minimum contenant un des substrat(s) {Ai} ; et
o) caractériser le ou les gènes codant pour l'enzyme ou les enzymes impliquée(s) dans la conversion du substrat {Ai} en produit {B} dans ladite ou lesdites cellule(s) receveuse(s) transformées (Ai+ ; B+) sélectionnée(s) à l'étape n).

8. Méthode selon la revendication 7, comprenant, avant l'étape m), tester ladite ou lesdites cellule(s) hôte(s) (Ai- ; B+) transformée(s) sur un milieu minimum contenant plusieurs substrat(s) {Ai} comme seule source d'un élément essentiel à la croissance et sélectionner ladite ou lesdites cellule(s) hôte(s) capable(s) de croissance sur ledit milieu minimum contenant plusieurs substrat(s) {Ai}; ladite ou lesdites cellule(s) hôte(s) sélectionnée(s) étant ensuite soumise à l'étape m) et suivantes.

9. Méthode selon la revendication 7 ou 8, dans laquelle :
- entre les étapes k) et 1), ladite ou lesdites cellule(s) hôte(s) (Ai- ; B+) est/sont modifiée(s) par le remplacement du premier marqueur de sélection présent dans le vecteur contenant la séquence d'acide nucléique introduite à l'étape b) par un nouveau marqueur de sélection ;
- ladite banque de séquences d'acide nucléique de l'étape 1) comprend un marqueur de sélection différent de celui porté par ladite ou lesdites cellule(s) hôte(s) (Ai-B+)
- la méthode comprend en outre les étapes suivantes :
kk) l'extraction et la purification des vecteurs contenus dans ladite ou lesdites cellule(s) hôte(s) sélectionnée(s) à l'étape k) ;
kkk) la mutagenèse in vitro dudit vecteur purifié à l'étape kk), avantageusement par transposition avec un élément transposable portant un résistance fonctionnelle à un antibiotique différente de celle pré-existente sur ce vecteur.
kkkk)) la transformation de ladite ou lesdites cellule(s) hôte(s) (Ai- ;B-) incapable(s) de métaboliser ledit ou lesdits substrats {Ai} et ledit produit {B} par les acides niucleiques mutés obtenus à l'étape précédente ;
kkkkk) la sélection des cellules hôtes transformées contenant uniquement ledit deuxième marqueur de sélection ; ces cellules transformées, de phénotype (Ai-B+), appelées cellules receveuses, font alors l'objet de la transformation décrite en l'étape 1).

10. Méthode selon l'une quelconque des revendications précédentes, dans laquelle lesdites cellules hôtes sont des cellules eucaryotes ou procaryotes.

11. Méthode selon la revendication 10, dans laquelle lesdites cellules hôtes sont :
- cultivables dans des conditions standards connus de l'homme de l'art,
- transformables, et
- capables de maintenir de façon stable les ADN exogènes transformants.

12. Méthode selon la revendication 10 ou 11, dans laquelle lesdites cellules hôtes sont des bactéries.

13. Méthode selon l'une quelconque des revendications précédentes, dans laquelle ladite banque de séquences d'acide nucléique est une banque metagénomique.

14. Méthode selon l'une quelconque des revendications 1 à 12, dans laquelle ladite banque de séquences d'acide nucléique est issue d'organismes procaryotes ou eucaryotes cultivables.

15. Méthode selon l'une quelconque des revendications 1 à 12, dans laquelle ladite banque de séquence d'acide nucléique est issue d'organismes procaryotes ou eucaryotes non-cultivables.

16. Méthode de sélection ou de préparation de cellule hôte (Ai- ; 13-) incapable de métaboliser ledit ou lesdits substrats {Ai} et ledit produit {B} comprenant les étapes suivantes :
- tester une population de cellules hôtes, cultivables dans des conditions standards de laboratoire et dans des conditions industrielles de production, transformables, et capables de maintenir de façon stable les ADN exogènes transformants, sur un milieu minimum contenant le(s) substrat(s) {Ai} et ledit produit {B} comme seule source d'un élément essentiel à la croissance; et,
- sélectionner la ou les cellule(s) hôte(s) incapable(s) de croissance sur ledit milieu minimum contenant le(s) substrat(s) {Ai} et ledit produit {B}.

## Claims

1. A method for selecting or preparing cells comprising at least one metabolic pathway or metabolic pathway family enabling the transformation of one or more substrate(s) {Ai} into a desired product {B}, comprising the following steps :
a) providing a population of host cells (Ai- ; B-) incapable of metabolising said substrate or substrates {Ai} and said product {B} ;
b) transforming said population of host cells with a library of sequences of nucleic acid ;
c) testing in parallel said population of transformed host cells on minimum media containing either one of the substrates {Ai}, or said product {B} as the only source of an element essential to growth; and,
d) selecting said host cell(s) capable of growth on a minimum medium containing one of the substrates {Ai} and on a minimum medium containing said product {B} (Ai+ ; B+).

2. The method according to Claim 1, comprising, before step c), a step consisting of testing said population of transformed host cells on a minimum medium containing the substrate(s) {Ai} and said product {B} as the only source of an element essential to growth and selecting said host cell(s) capable of growth on said minimum medium containing the substrate(s) {Ai} and said product {B}; said selected host cell(s) then being subjected to step c) and the subsequent steps.

3. The method according to Claim 1 or 2, comprising, after step d), the following steps :
e) implementing *in vitro* mutagenesis of the molecule of nucleic acid isolated from said transformed host cell(s) (Ai+ ; B+) in step d) ;
f) re-transforming the population of host cells (Ai- ; B-) described in step a) with the population of nucleic acids mutated *in vitro* in step e) and testing the host cell(s) thus transformed on minimum media containing either one of the substrate(s) {Ai}, or said product {B} as the only source of an element essential to growth; and,
g) selecting said transformed host cell(s) incapable of growth on a minimum medium containing one of the substrate(s) {Ai} and capable of growth on a minimum medium containing said product {B} (Ai- ; B+), and optionally isolating the mutated molecule of nucleic acid.

4. The method according to Claim 3, comprising the characterisation of the gene or genes encoding the enzyme or enzymes involved in the conversion of the substrate {Ai} into product {B} in said host cell(s) (Ai- ; B+) selected in step g).

5. The method according to Claim 3, comprising, after step f), in parallel to step g):
h) selecting said transformed host cell(s) which has (have) become incapable of growth on a minimum medium containing one of the substrate {Ai} and on a minimum medium containing said product {B} (Ai- ; B-) ;
i) implementing a quantitative analysis of the accumulation of the product {B} of said transformed host cells(s) (Ai- ; B-) on a rich medium supplemented by {Ai} ; and
j) selecting said transformed host cell(s) (Ai- ; B-) accumulating the product {B} on a rich medium and optionally isolating in parallel the mutated molecule of nucleic acid introduced during the transformation of step f).

6. The method according to Claim 5, comprising the characterisation of the gene or genes encoding the enzyme or enzymes involved in the conversion of the substrate {Ai} into product {B} in said host cell(s) (Ai- ; B-) selected in step j).

7. The method according to any of the preceding claims, comprising, after step c), in parallel 1 to step d) and the subsequent steps, the following steps :
k) selecting said transformed host cell(s), incapable of growth on a minimum medium containing one of the substrates {Ai} and capable of growth on a minimum medium containing said product {B}, called receiving cell(s) (Ai- ; B+) ;
l) transforming said receiving cell(s) (Ai- ; B+) with a library of sequences of nucleic acid;
m) testing in parallel said transformed receiving cell(s) (Ai- ; B+) on a minimum medium containing one of the substrate(s) {Ai} ; and
n) selecting said transformed receiving cell(s) capable of growth on a minimum medium containing one of the substrates {Ai} ; and
o) characterising the gene or genes encoding the enzyme or enzymes involved in the conversion of the substrate {Ai} into product {B} in said transformed receiving cell(s) (Ai+ ; B+) selected in step n).

8. The method according to Claim 7, comprising, before step m), testing said host cell(s) (Ai-; B+) transformed on a minimum medium containing several substrates {Ai} as the only source of an element essential to growth and selecting said host cell(s) capable of growth on said minimum medium containing several substrates {Ai}; said selected host cell(s) then being subjected to step m) and the subsequent steps.

9. The method according to Claim 7 or 8, wherein:
- between steps k) and 1), said host cell(s) (Ai- ; B+) is/are modified by replacing the first selection marker present in the vector containing the sequence of nucleic acid introduced in step b) with a new selection marker ;
- said library of sequences of nucleic acid from step 1) includes a selection marker different to that carried by said host cell(s) (Ai-B+)
- the method further includes the following steps :
kk) the extraction and purification of the vectors contained in said host cell(s) selected in step k) ;
kkk) the *in vitro* mutagenesis of said vector purified in step kk), advantageously by transposition with a transposable element carrying a functional resistance to an antibiotic different to that previously existing on this vector.
kkkk) the transformation of said host cell(s) (Ai- ;B-) incapable of metabolising said substrate(s) {Ai} and said product {B} by the mutated nucleic acids obtained in the previous step ;
kkkkk) the selection of transformed host cells containing just said second selection marker ; these transformed cells, ofphenotype (Ai-B+), called receiving cells, are then the object of the transformation described in step 1).

10. The method according to any of the preceding claims, wherein said host cells are eukaryotic or prokaryotic cells.

11. The method according to Claim 10, wherein said host cells are :
- cultivable under standard conditions known by the man skilled in the art,
- transformable, and
- capable of stably maintaining the transforming exogenous DNA.

12. The method according to Claim 10 or 11, wherein said host cells are bacteria.

13. The method according to any of the preceding claims, wherein said library of sequences of nucleic acid is a metagenomic library.

14. The method according to any of Claims 1 to 12, wherein said library of nucleic acid sequences originates from cultivatable prokaryotic or eukaryotic organisms.

15. The method according to any of Claims 1 to 12, wherein said library of nucleic acid sequences originates from non-cultivatable prokaryotic or eukaryotic organisms.

16. A method for selecting or preparing a host cell (Ai- ; B-) incapable of metabolising said substrate(s) {Ai} and said product {B} comprising the following steps :
- testing a population of host cells, cultivatable under standard laboratory conditions and under industrial production conditions, transformable, and capable of stably maintaining the transforming exogenous DNA, on a minimum medium containing the substrate(s) {Ai} and said product {B} as the only source of an element essential to growth; and,
- selecting the host cell(s) incapable of growth on said minimum medium containing the substrate(s) {Ai} and said product {B}.

## Patentansprüche

1. Verfahren zur Selektion oder Präparation von Zellen, umfassend wenigstens einen Stoffwechselweg oder eine Familie von Stoffwechselwegen, der/die die Umwandlung eines oder mehrerer Substrate {Ai} in ein erwünschtes Produkt {B} erlaubt, wobei das Verfahren die folgenden Schritte umfasst:
a) Bereitstellen einer Population an Wirtszellen (Ai-; B-), die nicht die Fähigkeit besitzen, besagtes oder besagte Substrate {Ai} und besagtes Produkt {B} zu metabolisieren;
b) Transformieren besagter Population an Wirtszellen mit einer Bank von Nukleinsäuresequenzen;
c) paralleles Testen besagter Population transformierter Wirtszellen auf Minimalmedien, die entweder eines der Substrate {Ai} oder besagtes Produkt {B} als einzige Quelle eines für das Wachstum essentiellen Bestandteils enthalten; und
d) Selektieren besagter Wirtszelle oder Wirtszellen, die die Fähigkeit besitzt/besitzen, auf einem eines der Substrate zeit enthaltenden Minimalmedium und auf einem besagtes Produkt {B} enthaltenden Minimalmedium zu wachsen (Ai+; B+).

2. Verfahren gemäß Anspruch 1, umfassend, vor Schritt c), einen Schritt, bestehend aus Testen einer Population transformierter Wirtszellen auf einem Minimalmedium, das das/die Substrat(e) {Ai} und besagtes Produkt {B} als einzige Quelle eines für das Wachstum essentiellen Bestandteils enthält, und Selektieren besagter Wirtszelle oder Wirtszellen, die die Fähigkeit besitzt/besitzen, auf besagtem Minimalmedium, das das/die Substrat(e) {Ai} und besagtes Produkt {B} enthält, zu wachsen; wobei besagte selektierte Wirtszelle oder Wirtszellen anschließend dem Schritt c) und folgenden unterzogen wird/werden.

3. Verfahren gemäß Anspruch 1 oder 2, umfassend, nach Schritt d), die folgenden Schritte:
e) Durchführen einer *in vitro* Mutagenese des Nukleinsäuremoleküls, das von besagter oder besagten in Schritt d) transformierten Wirtszellen (Ai+; B+) isoliert ist;
f) Retransformieren der in Schritt a) beschriebenen Population an Wirtszellen (Ai-; B-) mit der Population der in Schritt e) *in vitro* mutierten Nukleinsäuren und Testen der so transformierten Wirtszelle oder Wirtszellen auf Minimalmedien, die entweder eines der Substrate {Ai} oder besagtes Produkt {B} als einzige Quelle eines für das Wachstum essentiellen Bestandteils enthalten; und
g) Selektieren besagter transformierter Wirtszelle oder Wirtszellen (Ai-; B+), die nicht die Fähigkeit besitzt/besitzen, auf einem eines der Substrate {Ai} enthaltenden Minimalmedium zu wachsen, und die Fähigkeit besitzt/besitzen, auf einem besagtes Produkt {B} enthaltenden Minimalmedium zu wachsen, und anschließend gegebenenfalls Isolieren des mutierten Nukleinsäuremoleküls.

4. Verfahren gemäß Anspruch 3, umfassend die Charakterisierung des oder der Gene, die für das Enzym oder die Enzyme codieren, die an der Umwandlung des Substrats {Ai} in Produkt {B} in besagter oder besagten in Schritt g) selektierten Wirtszellen (Ai-; B+) beteiligt sind.

5. Verfahren gemäß Anspruch 3, umfassend, nach Schritt f), parallel zu Schritt g):
h) Selektieren besagter transformierter Wirtszelle oder Wirtszellen (Ai-; B-), die die Fähigkeit verloren hat/haben, auf einem eines der Substrate {Ai} enthaltenden Minimalmedium und auf einem besagtes Produkt {B} enthaltenden Minimalmedium zu wachsen;
i) Durchführen einer quantitativen Analyse der Akkumulation des Produkts {B} von besagter oder besagten transformierten Wirtszellen (Ai-; B-) auf einem mit {Ai} supplementierten Vollmedium; und
j) Selektieren besagter transformierter Wirtszelle oder Wirtszellen (Ai-; B-), die das Produkt {B} auf einem Vollmedium akkumulieren, und gegebenenfalls paralleles Isolieren des mutierten Nukleinsäuremoleküls, das bei der Transformation in Schritt f) eingeführt worden ist.

6. Verfahren gemäß Anspruch 5, umfassend die Charakterisierung des oder der Gene, die für das Enzym oder die Enzyme codieren, die an der Umwandlung des Substrats {Ai} in Produkt {B} in besagter oder besagten in Schritt j) selektierten Wirtszellen (Ai-; B-) beteiligt sind.

7. Verfahren gemäß einem der vorhergehenden Ansprüche, umfassend, nach Schritt c), parallel zu Schritt d) und folgenden, die folgenden Schritte:
k) Selektieren besagter transformierter Wirtszelle oder Wirtszellen, die nicht die Fähigkeit besitzt/besitzen, auf einem eines der Substrate {Ai} enthaltenden Minimalmedium zu wachsen, und die Fähigkeit besitzt/besitzen, auf einem besagtes Produkt {B} enthaltenden Minimalmedium zu wachsen, und als Empfängerzelle oder Empfängerzellen (Ai-; B+) bezeichnet wird/werden;
l) Transformieren besagter Empfängerzelle oder Empfängerzellen (Ai-; B+) mit einer Bank von Nukleinsäuresequenzen;
m) paralleles Testen besagter transformierter Empfängerzelle oder Empfängerzellen (Ai-; B+) auf einem eines der Substrate {Ai} enthaltenden Minimalmedium; und
n) Selektieren besagter transformierter Empfängerzelle oder Empfängerzellen, die die Fähigkeit besitzt/besitzen, auf einem eines der Substrate {Ai} enthaltenden Minimalmedium zu wachsen; und
o) Charakterisieren des oder der Gene, die für das Enzym oder die Enzyme codieren, die an der Umwandlung des Substrats zeit in Produkt {B} in besagter oder besagten in Schritt n) selektierten transformierten Empfängerzellen (Ai+; B+) beteiligt sind.

8. Verfahren gemäß Anspruch 7, umfassend, vor Schritt m), Testen besagter transformierter Wirtszelle oder Wirtszellen (Ai-; B+) auf einem Minimalmedium, das mehrere Substrate {Ai} als einzige Quelle eines für das Wachstum essentiellen Bestandteils enthält, und Selektieren besagter Wirtszelle oder Wirtszellen, die die Fähigkeit besitzt/besitzen, auf besagtem mehrere Substrate {Ai} enthaltendem Minimalmedium zu wachsen; wobei besagte selektierte Wirtszelle oder Wirtszellen anschließend dem Schritt m) und folgenden unterzogen wird/werden.

9. Verfahren gemäß Anspruch 7 oder 8, in dem:
- zwischen den Schritten k) und 1) besagte Wirtszelle oder Wirtszellen (Ai-; B+) durch den Austausch des ersten Selektionsmarkers, der in dem Vektor vorhanden ist, der die in Schritt b) eingeführte Nukleinsäuresequenz enthält, durch einen neuen Selektionsmarker modifiziert wird/werden;
- besagte Bank von Nukleinsäuresequenzen von Schritt 1) einen Selektionsmarker umfasst, der sich von demjenigen unterscheidet, der von besagter oder besagten Wirtszellen (Ai-; B+) getragen wird;
- das Verfahren außerdem die folgenden Schritte umfasst:
kk) Extraktion und Reinigung der Vektoren, die in besagter oder besagten in Schritt k) selektierten Wirtszellen enthalten sind;
kkk) in vitro Mutagenese des in Schritt kk) gereinigten besagten Vektors, vorteilhafterweise durch Transposition mit einem Transposonelement, das eine funktionelle Resistenz gegen ein Antibiotikum trägt, die sich von der bei diesem Vektor bereits vorhandenen Resistenz unterscheidet;
kkkk) Transformation besagter Wirtszelle oder Wirtszellen (Ai-; B-), die nicht die Fähigkeit besitzt/besitzen, besagtes oder besagte Substrate {Ai} und besagtes Produkt {B} zu metabolisieren, mit den im vorhergehenden Schritt erhaltenen mutierten Nukleinsäuren;
kkkkk) Selektion der transformierten Wirtszellen, die nur besagten zweiten Selektionsmarker enthalten; diese transformierten Zellen des Phänotyps (Ai-; B+), die als Empfängerzellen bezeichnet werden, sind nun Gegenstand der in Schritt 1) beschriebenen Transformation.

10. Verfahren gemäß einem der vorhergehenden Ansprüche, in dem besagte Wirtszellen eukaryotische oder prokaryotische Zellen sind.

11. Verfahren gemäß Anspruch 10, in dem besagte Wirtszellen:
- unter dem Fachmann bekannten Standardbedingungen kultivierbar sind,
- transformierbar sind, und
- die Fähigkeit besitzen, transformierte exogene DNA stabil zu behalten.

12. Verfahren gemäß Anspruch 10 oder 11, in dem besagte Wirtszellen Bakterien sind.

13. Verfahren gemäß einem der vorhergehenden Ansprüche, in dem besagte Bank von Nukleinsäuresequenzen eine metagenomische Bank ist.

14. Verfahren gemäß einem der Ansprüche 1 bis 12, in dem besagte Bank von Nukleinsäuresequenzen von kultivierbaren prokaryotischen oder eukaryotischen Organismen stammt.

15. Verfahren gemäß einem der Ansprüche 1 bis 12, in dem besagte Bank von Nukleinsäuresequenzen von nichtkultivierbaren prokaryotischen oder eukaryotischen Organismen stammt.

16. Verfahren zur Selektion oder Präparation einer Wirtszelle (Ai-; B-), die nicht die Fähigkeit besitzt, besagtes oder besagte Substrate {Ai} und besagtes Produkt {B} zu metabolisieren, umfassend die folgenden Schritte:
- Testen einer Population an Wirtszellen, die unter standardisierten Laborbedingungen und unter industriellen Produktionsbedingungen kultivierbar, transformierbar sind und die Fähigkeit besitzen, transformierte exogene DNA stabil zu behalten, auf einem Minimalmedium, das das/die Substrat(e) {Ai} und besagtes Produkt {B} als einzige Quelle eines für das Wachstum essentiellen Bestandteils enthält; und
- Selektieren der Wirtszelle oder Wirtszellen, die nicht die Fähigkeit besitzt/besitzen, auf besagtem Minimalmedium zu wachsen, das das/die Substrat(e) {Ai} und besagtes Produkt {B} enthält.
